(19) Europäisches Patentamt
European Patent Office
Office européen des brevets

(11) Veröffentlichungsnummer: **0 284 552 A1**

# (12) EUROPÄISCHE PATENTANMELDUNG

(21) Anmeldenummer: **88810120.1**

(22) Anmeldetag: **26.02.88**

(51) Int. Cl.[4]: **C 07 D 498/08**
A 61 K 31/395
//(C07D498/08,307:00,267:00)

(30) Priorität: **06.03.87 CH 848/87**

(43) Veröffentlichungstag der Anmeldung:
**28.09.88 Patentblatt 88/39**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Traxler, Peter, Dr.**
**Bündtenring 5**
**CH-4124 Schönenbuch (CH)**

**Lydon, Nicholas, Dr.**
**Bollwerkstrasse 30c**
**CH-4102 Binningen (CH)**

## (54) 4-Benzyl-piperazinyl-Hydrazone.

(57) Antiviral wirksame Hydrazone, die sich von einem 3-Formylrifamycin SV oder S, oder einem in Stellungen 16,17,18,19 und gegebenenfalls auch 28,29 gesättigten Analogen davon, als Aldehyd-Komponente einerseits und andererseits von einem substituierten 1-Amino-4-benzyl-piperazin der Formel

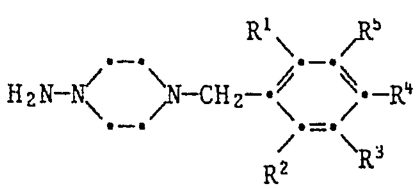

ableiten, worin jeder der Reste $R^1$, $R^2$, $R^3$, $R^4$ und $R^5$ zusammen mit seinem benachbarten Rest einen zweiwertigen aliphatischen Kohlenwasserstoffrest mit 3-10 C-Atomen bedeuten kann, welcher gemeinsam mit den entsprechenden 2 C-Atomen des zentralen Phenylrings einen anelierten 5- oder 6-gliedrigen carbocyclischen Ring bildet, wobei die übrigen Reste Wasserstoff oder $C_{1-4}$-Alkyl bedeuten, oder worin $R^4$ einen $C_{1-12}$-Alkyl, Phenyl oder Wasserstoff und $R^1$, $R^2$, $R^3$ und $R^5$ unabhängig je ein $C_{1-4}$-Alkyl oder Wasserstoff bedeuten, wobei mindestens einer aller Reste vom Wasserstoff verschieden sein muss, sowie ihre Salze zeichnen sich als Hemmer der reversen Transkriptase aus und können dementsprechend zur Behandlung von Krankheiten, bei welchen diese von Bedeutung ist, wie AIDS, angewendet werden. Diese Verbindungen sind durch allgemein bekannte Analogieverfahren zugänglich.

EP 0 284 552 A1

**Beschreibung**

4-Benzyl-piperazinyl-Hydrazone

Gegenstand der vorliegenden Erfindung sind neue antiviral wirksame Hydrazone, die sich von einem im Phenylring substituierten 1-Amino-4-benzyl-piperazin als Hydrazin-Komponente und 3-Formylrifamycin SV oder S, oder einem in Stellungen 16,17,18,19 und gegebenenfalls auch 28,29 gesättigten Analogen davon, als Aldehyd-Komponente ableiten, und welche der Formel

Rif-CH=N-W (I)

entsprechen, worin Rif einen Rest der Teilformel

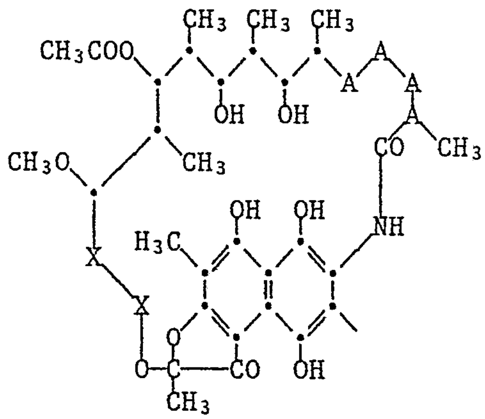

[Rif SV]

oder

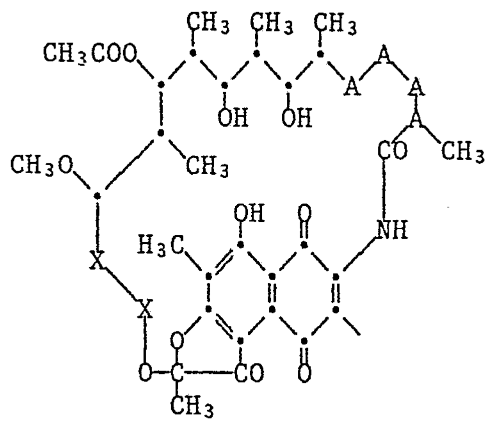

[Rif S]

darstellt, in welchem A-A-A-A Buta-1,3-dien-1,4-diyl und X-X Vinylen oder A-A-A-A Tetramethylen und X-X Ethylen oder Vinylen ist, und worin W einen Piperazinyl-Rest der Teilformel

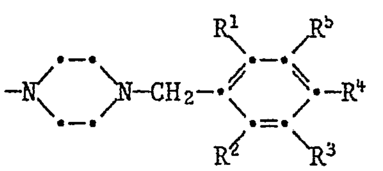

(W)

darstellt, worin jeder der Reste $R^1$, $R^2$, $R^3$, $R^4$ und $R^5$ zusammen mit seinem benachbarten Rest einen zweiwertigen aliphatischen Kohlenwasserstoffrest mit 3-10 C-Atomen bedeuten kann, welcher gemeinsam mit den entsprechenden 2 C-Atomen des zentralen Phenylrings einen anellierten 5-oder 6-gliedrigen carbocyclischen Ring bildet, wobei die übrigen Reste Wasserstoff oder $C_{1-4}$-Alkyl bedeuten, oder worin $R^4$ einen $C_{1-12}$-Alkyl, Phenyl oder Wasserstoff und $R^1$, $R^2$, $R^3$ und $R^5$ unabhängig je ein $C_{1-4}$-Alkyl oder Wasserstoff bedeuten, wobei mindestens einer aller Reste vom Wasserstoff verschieden sein muss, sowie ihre Salze.

Die Erfindung betrifft ferner auch Verfahren zur Herstellung der Verbindungen der Formel I einschliesslich ihrer Salze, diese enthaltende pharmazeutische Präparate, sowie die Verwendung dieser Verbindungen und Präparate.

Infolge der sehr engen Beziehung zwischen 1,4-Chinon- und 1,4-Hydrochinon-Form (entsprechend Rifamycin-S und -SV) und der Leichtigkeit, mit welcher die beiden Formen ineinander übergehen, sind überall, wo nicht spezifisich anders angegeben, beide Formen im Gegenstand der Erfindung inbegriffen, wobei jedoch die SV-Form als die bevorzugte anzusehen ist.

Infolge der sehr engen Beziehung zwischen der syn- und anti-Konfiguration der Aldimino-Doppelbindung -CH=N- wird zwischen den entsprechenden stereoisomeren Hydrazonen (sowie Gemischen davon) kein Unterschied gemacht, alle Formen sind gleichsam im Gegenstand der Erfindung inbegriffen. Bei Benennung der erfindungsgemässen Verbindungen wird dem Namen "Hydrazon" von der alternativen Bezeichnung "Aldimin" Vorzug gegeben, da der erstere die chemischen Eigenschaften dieser Verbindungsklasse genauer beschreibt.

In Verbindungen der Formel I, worin in der Teilformel (W) jeder der Reste $R^1$, $R^2$, $R^3$, $R^4$ und $R^5$ zusammen mit seinem benachbarten Rest einen zweiwertigen aliphatischen Kohlenwasserstoffrest mit 3-10 C-Atomen bedeuten kann, welcher gemeinsam mit den entsprechenden 2 C-Atomen des zentralen Phenylrings einen

anellierten 5- oder 6-gliedrigen carbocyclischen Ring bildet, wobei die übrigen Reste Wasserstoff oder $C_{1-4}$-Alkyl bedeuten, stehen beispielsweise die Reste $R^2$ und $R^3$ oder $R^3$ und $R^4$, ferner $R^2$ und $R^3$ sowie zusätzlich $R^4$ und $R^5$ bzw. $R^1$ und $R^5$ jeweils für einen entsprechenden zweiwertigen aliphatischen Kohlenwasserstoffrest.

Ein zweiwertiger aliphatischer Kohlenwasserstoffrest mit 3-10 C-Atomen ist geradkettig, ferner verweigt, hat 3 oder 4 C-Atome in gerader Kette und kann eine oder 2 Doppelbindungen aufweisen und steht insbesondere für Buta-1,3-dien-1,4-diyl, ferner Tri- oder Tetramethylen. Der entsprechend gebildete anellierte Ring kann einen oder mehrere, wie zwei, $C_{1-4}$-Alkylreste tragen oder vorzugsweise unsubstituiert sein. Als ein derart anellierter Ring ist der Benzo-Ring, insbesondere ein unsubstituierter, besonders bevorzugt.

Ein $C_{1-12}$-Alkyl ist z.B. ein lineares Alkyl, beispielsweise mit 5-12 C-Atomen, und insbesondere ein $C_{1-4}$-Alkyl, wie Ethyl, Propyl, i-Propyl, n-Butyl, Isobutyl oder tert-Butyl, in erster Linie aber Methyl.

Einen bevorzugten Gegenstand der vorliegenden Erfindung stellen die Verbindungen der Formel I dar, in welchen im Rest W die Symbole $R^1$ und $R^2$ unabhängig je ein $C_{1-4}$-Alkyl, $R^3$ und $R^5$ unabhängig je Wasserstoff oder $C_{1-4}$-Alkyl und $R^4$ Phenyl oder $C_1$-$C_{12}$-Alkyl bedeuten, oder $R^2$ zusammen mit $R^3$ oder $R^3$ zusammen mit $R^4$ ein gegebenenfalls durch $C_{1-4}$-Alkyl substituiertes Buta-1,3-dien-1,4-diyl, Trimethylen oder Tetramethylen darstellen, $R^1$ und $R^5$ zusammen eine dieser Bedeutungen haben oder jedes einzeln Wasserstoff oder $C_{1-4}$-Alkyl bedeuten, wobei $R^4$ bzw. $R^2$ Wasserstoff oder $C_{1-4}$-Alkyl bedeuten, sowie ihre Salze. Besonders bevorzugt sind Verbindungen der Formel I, worin im Rest W die Symbole $R^1$ und $R^2$ je Methyl oder, falls $R^4$ Phenyl darstellt, auch Wasserstoff, $R^4$ Wasserstoff, Phenyl, ein lineares $C_{5-12}$-Alkyl oder $C_{1-4}$-Alkyl, insbesondere Methyl oder tert-Butyl, und $R^3$ und $R^5$ Wasserstoff bedeuten, oder worin die Symbole $R^2$ und $R^3$ zusammen, oder $R^3$ und $R^4$ zusammen Buta-1,3-dien-1,4-diyl, $R^1$ und $R^5$ entweder zusammen Buta-1,3-dien-1,4-diyl oder jedes einzeln Wasserstoff oder Methyl, und $R^4$ bzw. $R^2$ Wasserstoff darstellen, sowie Salze, insbesondere pharmazeutisch verwendbare Salze davon.

Ganz besonders betrifft die Erfindung Verbindungen der Formel I, worin Rif für den Rest [Rif SV] steht, in welchem X-X vorzugsweise Vinylen und A-A-A-A Buta-1,3-dien-1,4-diyl oder Tetramethylen darstellt, und im Rest W die Symbole $R^1$ und $R^2$ $C_{1-4}$-Alkyl, insbesondere Methyl, $R^4$ Wasserstoff oder $C_{1-4}$-Alkyl, insbesondere Methyl oder tert-Butyl, und $R^3$ und $R^5$ Wasserstoff bedeuten, oder worin die Reste $R^2$ und $R^3$ zusammen, ferner auch $R^3$ und $R^4$, zusammen Buta-1,3-dien-1,4-diyl, $R^1$ und $R^5$ entweder zusammen Buta-1,3-dien-1,4-diyl oder jedes einzeln Wasserstoff und $R^4$ bzw. $R^2$ Wasserstoff bedeuten, oder aber worin $R^4$ Phenyl und $R^1$, $R^2$, $R^3$ und $R^5$ unabhängig Methyl oder vor allem Wasserstoff bedeuten, sowie ihre Salze, insbesondere pharmazeutisch verwendbare Salze mit basen.

Unter den Verbindungen der Formel I mit der oben angegebenen bevorzugten Bedeutungen von Rif sind vor allem diejenigen hervorzuheben, worin der Rest W für 4-(2,4,6-Trimethylbenzyl)-piperazinyl, 4-(2,6-Dimethyl-4-tert-butyl-benzyl)-piperazinyl, 4-(1-Naphthylmethyl)-piperazinyl, 4-(2-Naphthylmethyl)-piperazinyl, 4-(9-Anthrylmethyl)-piperazinyl, sowie 4-(4-Biphenylylmethyl)-piperazinyl steht. Bevorzugt sind auch übrige Verbindungen der Formel I, welche diese Reste tragen.

Vor allem betrifft die Erfindung die in der Beschreibung und insbesondere in den Beispielen genannten Verbindungen.

In 4-Stellung des Piperazinyl-Rests substituierte Hydrazone, abgeleitet von 1-Aminopiperazin mit 3-Formylrifamycin SV oder S (nicht aber mit entsprechenden 16,17,18,19-Tetrahydro- und 16,17,18,19,28,29-Hexahydro-Analogen) wurden allgemein bereits in Betracht gezogen. So wurden z.B. im U.S. Patent 3,342,810 unter anderen antimikrobiell wirksamen Derivaten des 3-Formylrifamycins SV auch Hydrazone der Teilformel $=N-N(R_2)R_3$ beschrieben und beansprucht, worin $R_2$ und $R_3$ (neben einer Reihe von individuellen Bedeutungen für jedes einzelne Symbol) beide gemeinsam und zusammen mit dem Stickstoffatom für einen heterocyclischen Ring mit 5-7 Atomen stehen. Ausser dieser Definition im Patentanspruch 6 ist keine nähere Charakterisierung des heterocyclischen Ringes in der Beschreibung zu finden, lediglich sind 9 Beispiele spezifischer Einzelverbindungen (einschliesslich biologischer Wirkungsdaten) in der Tabelle, Spalte 3, Verbindungen III-XIV, angeführt. Von diesen wird im weiteren Text (Spalte 5, Zeilen 64-75, und Spalte 6, Zeilen 30-45) das Hydrazon von 3-Formylrifamycin SV mit 1-Amino-4-methylpiperazin wegen seiner hervorragenden antituberkulösen Wirksamkeit hervorgehoben und im spezifischen Anspruch 15 geschützt. Diese Verbindung gehört bis jetzt, unter dem generischen Namen Rifampicin, zu den führenden Therapeutika zur Bekämpfung von Tuberkulose. Hydrazone mit einem 4-Benzyl-piperazinyl-Rest sind lediglich durch das 3-(4-Benzyl-2,6-dimethylpiperazinyliminomethyl)-rifamycin SV, d.h. Verbindung XIII in Spalte 3 der Tabelle, vertreten, die aber weder in der Beschreibung noch in den Ansprüchen erwähnt ist. Die beschriebenen Verbindungen weisen eine sehr gute antituberkulöse Wirkung sowie eine deutliche antibakterielle Wirkung auf und wurden deshalb in entsprechenden Indikationen, insbesondere als Tuberkulostetika, für medizinische Anwendung vorgeschlagen. Die Möglichkeit irgendeiner antiviralen oder tumorhemmenden Wirkung solcher Verbindungen wurde nie in irgendeiner Form erwähnt oder in Betracht gezogen.

Dagegen wurde nun gefunden, dass sich die eingangs definierten neuen Verbindungen der Formel I zwar durch die übliche antituberkulöse Wirksamkeit auch auszeichnen, dabei aber überraschenderweise in erster Linie die reverse Transkriptase, ein für Retroviren oder Oncornaviren (wie RNS-Tumor- und Leukämie-Viren) charakteristisches Enzym, hemmen; Retroviren benötigen dieses Enzym für ihren natürlichen Replikationscyclus [Baltimore: Nature, 226, 1209 (1970); sowie Temin und Mizutani: Nature, 226, 1211 (1970)].

Der Nachweis von Erkrankungen, die durch Retroviren verursacht werden, basiert z.B. auf den folgenden

Befunden: Der Nachweis von Typ C-Retroviren und deren reversen Transkriptase beim Menschen erfolgte zuerst bei einer T-Zell Leukaemie; das Virus wurde Human T-cell leukaemia virus (HTLV-I) genannt. Bei weiteren T-Zellen-Leukämien und -Lymphomen wurden die gleichen Viren und ein ähnliches Retrovirus (HTLV-II) gefunden [Gallo: Cancer Surveys (Ed. Franks, Wyke und Weiss), Bd. 3, S. 113-160; Oxford Univ. Press, (1984)]. Schliesslich wurde ein derartiges Virus auch im Zusammenhang mit AIDS (Acquired Immune Deficiency Syndrome)-Erkrankungen isoliert [Gottlieb et al.: Morbid. Mortal. Weekly Rep., 30, 25,-(1981); Friedman-Kien et al.: Morbid. Mortal. Weekly Rep., 30, 305 (1981); Gottlieb et al.: New Engl. J. Med., 305, 1425 (1981); Masur: New Engl. J. Med., 305, 1431 (1981); Siegal et al.: New Engl. J. Med., 305, 1439 (1981); CDC Task Force on Kaspoi's Sarcoma and Opportunistic Infections, New Engl. J. Med., 306, 248 (1982)]; dieses wurde anfänglich mit HTLV-III oder LAV bezeichnet [Essex et al.: Science, 220, 859 (1983), Gelmann et al.: Science, 220, 862 (1983); Gallo et al.: Science, 220, 865 (1983); Gallo: Cancer Surverys, 3, 113 (1984); Barré-Sinoussi et al.: Science 220, 868 (1983); Hirsch und Levy: Viruses in Human Malignancy and AIDS, ASCO/AACR Symposioum, May 1984, Toronto]. Die heutige gültige Bezeichnung für diese Gruppe von Viren lautet HIV.

Diese, der HTLV-Familie angehörenden Retroviren benötigen die reverse Transkriptase für die Bildung einer Doppelstrang-DNA (Provirus), welche in das Zell-Genom "integriert" wird und zur malignen Transformation führen kann [Strayer und Gillespie, The Nature and Organization of Retroviral Genes in Animal Cells. Virology Monographs, Bd. 17 (Springer Ed., 1980)].

Während nach der Induktion maligner leukämischer, lymphatischer oder tumoröser Neubildungen durch Retroviren eine Abnahme der reversen Transkriptase und des Virus HTLV-I und -II nachgewiesen werden kann und sekundäre, durch diese Viren induzierte Oncogene die Replikation der malignen Zellen steuern, ist das Virus HIV und die reverse Transkriptase bei AIDS nicht nur im Frühstadium, sondern während des ganzen Krankheitsverlaufs nachweisbar. Die laufende Infektion und die Funktionsstörung von immunkompetenten T-Helfer Zellen führt schliesslich zum Zusammenbruch des Immunosystems mit letalem Ausgang. Es ist anzunehmen, dass eine Hemmung der reversen Transkriptase und der Virusreplikation bei positivem Enzym-und Virusantigen-Nachweis [Beardsley: Nature, 311, 195 (1984)] in Risikopatienten, z.B. Homosexuellen, den Krankheitsprozess beeinflusst. Bei der Induktion von Leukämien und Lymphomen durch Retroviren (HTLV-I und HTLV-II), sowie möglicherweise auch bei durch Retroviren induzierten Sarkomen oder Mammarkarzinomen, sollte dagegen eine prophylaktische Anwendung möglich sein, sofern eine leicht und breit verfügbare diagnostische Erfassung von derartigen Risikopatienten vorausgesetzt werden kann.

Auch im Zusammenhang mit der nicht-A- und nicht-B-Hepatitis ist die reverse Transkriptase als spezifisches Enzym in Assoziation mit Viruspartikeln gefunden worden [Seto et al.: Lancet, 941 (1984)].

Die Verbindungen der vorliegenden Anmeldung können deshalb zur Prophylaxe und Therapie von Krankheiten, bei denen die reverse Transkriptase von Bedeutung ist, vor allem von durch Typ-C-Retroviren verursachten oder mitverursachten malignen Erkrankungen, aber auch von gewissen Immunkrankheiten und Autoimmunkrankheiten Verwendung finden. Als Tumorkrankheiten kommen in ersten Linie durch Retroviren verursachte Leukämien, Lymphome und Lymphosarkome, sowie möglicherweise auch Osteosarkome und Mammacarcinome in Betracht. Die Verbindungen der Erfindung eignen sich besonders auch zur Rezidivprophylaxe nach chirurgischer Therapie, Strahlentherapie oder zytostatischer oder antimetabolischer Chemotherapie. Als Immunkrankheit sei AIDS, als Autoimmunkrankheit systemischer Lupus erythematosus genannt, bei denen nach neueren Befunden RNS-Tumorviren auch eine wichtige Rolle zu spielen scheinen [Dennman: Med., Biol., 53, 61 (1975); Panem et al.: New England J. Med., 295, 470 (1976)].

Dabei weisen die Verbindungen gemäss der vorliegenden Erfindung eine grosse therapeutische Breite auf, indem sie eine signifikante Toxizität erst bei hohen Dosen, etwa in der Grössenordnung von 5000 mg/kg, aufweisen.

Die neuen Verbindungen können daher als Heilmittel, in erster Linie zur Behandlung von Infektionen, die durch Retroviren, wie z.B. AIDS-Viren oder Viren vom Typ HTLV I oder II, hervorgerufen werden, verwendet werden.

Die neuen erfindungsgemässen Verbindungen der Formel I (einschliesslich ihrer Salze) können mittels an sich bekannter allgemeiner Analogieverfahren hergestellt werden, z.B. indem man

a) ein 3-Formylrifamycin der Formel

Rif-CH=Z (II),

worin Rif die oben angegebene Bedeutung hat und Z eine freie oder funktionell abgewandelte Oxogruppe ist, mit einem N-Aminopiperazin der Formel

W-$NH_2$ (III),

worin W die oben angegebenen Bedeutungen hat, umsetzt oder

b) ein vom N'-unsubstituierten Aminopiperazin abgeleitetes Hydrazon der Formel

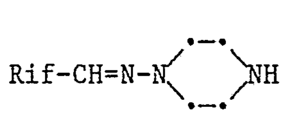

(IV),

worin Rif die oben angegebenen Bedeutungen hat, mit einer Verbindung der Formel

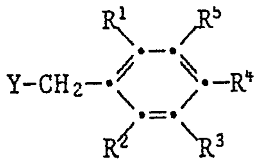

(V),

worin $R^1$, $R^2$, $R^3$, $R^4$ und $R^5$ die oben angegebenen Bedeutungen haben und Y für den Rest einer starken anorganischen oder organischen Säure steht, umsetzt, und gewünschtenfalls, wenn eine Verbindung der Formel I in der Chinon-Form erwünscht ist, eine in der Hydrochinon-Form vorliegende Verbindung der Formel I mit einem Oxidationsmittel behandelt, und/oder, wenn eine Verbindung der Formel I in der Hydrochinon-Form erwünscht ist, eine in der Chinon-Form vorliegende Verbindung der Formel I mit einem Reduktionsmittel behandelt und/oder eine in freier Form vorliegende Verbindung der Formel I in ein Salz davon überführt oder eine freie Verbindung der Formel I aus einem Salz davon freisetzt.

Die Umsetzung eines der Formel II entsprechenden 3-Formylrifamycins oder eines reaktionsfähigen funktionellen Derivats davon mit dem Hydrazin der Formel III erfolgt in an sich bekannter Weise, z.B. gemäss dem obengenannten U.S. Patent 3,342,810. Vornehmlich lässt man freies 3-Formylrifamycin SV oder ein teilweise gesättigtes Derivat davon der Formel II, worin Rif für [Rif SV] der obgenannten Bedeutung und Z für freies Oxo steht, und das N-Aminopiperazin (III) in etwa äquimolaren Mengen (oder einem kleinen Ueberschuss an diesem) und vorzugsweise in Anwesenheit eines organischen Lösungsmittels, wie eines Alkohols, z.B. Methanol, Ethanol oder Isopropylalkohol, eines offenkettigen oder cyclischen Ethers, z.B. Diethylether, 1,2-Dimethoxy- oder 1,2-Diethoxy-ethan, Tetrahydrofuran oder Dioxan, eines aliphatischen Esters oder Amids, z.B. Ethylacetat bzw. Dimethylformamid, ferner auch Dimethylsulfoxids und Acetonitrils, oder in einem Gemisch davon, bei Temperaturen von etwa -20° bis etwa 70°C, vorzugsweise zwischen Nullpunkt bis Raumtemperatur, reagieren. Die N-Aminopiperazin-Komponente (III) kann man auch in Form eines Säureadditionssalzes vorlegen und die Base erst in situ durch Zugabe eines basischen Hilfsstoffes, wie eines organischen Alkalimetallsalzes, z.B. Natrium- oder Kalium-acetat, oder einer tertiären organischen Base, wie eines tertiären Amins, z.B. Triethylamin, N-Methyl-oder N-ethyl-piperidin oder N-Methylmorpholin, oder aber einer heterocyclischen aromatischen Base vom Typ Pyridin und dessen Homologen oder Chinolin, freisetzen. Auch die Aldehyd-Komponente der Formel II kann in Form eines funktionellen Derivats vorliegen, z.B. als ein Alkalimetallsalz der Hydrochinon-Form des Ausgangsstoffes (II) mit freier Oxogruppe, oder insbesondere als ein reaktionsfähiges Derivat mit funktionell abgewandelter Aldehydgruppe, z.B. als eine Verbindung der Formel II, worin Rif die obengenannte Bedeutung hat und insbesondere [Rif S] ist und Z eine Oximino-, N-substituierte Imino-, unsubstituierte oder N-(mono-oder di)-substituierte Hydrazono-, oder Semicarbazono-Gruppe ist. Die Substituenten der Imino- und Hydrazono-Gruppe sind einwertige Kohlenwasserstoffreste je mit höchstens 8 C-Atomen, wie insbesondere Alkyl-oder Cycloalkyl-Reste mit höchstens 7 C-Atomen, Phenyl oder Benzyl, oder analoge zweiwertige Reste, welche zusammen mit dem Stickstoffatom einen gesättigten monocyclischen Heterocyclus mit 5-7 Ringgliedern bilden und gegebenenfalls einen zusätzlichen Heteroatom, wie Sauerstoff, Schwefel-(II), Stickstoff oder einfach mit $C_{1-4}$-Alkyl substituierten Stickstoff im Ring enthalten. Bevorzugt sind solche Substituenten und ihre Kombinationen, welche leicht flüchtige Amine bzw. Hydrazine ergeben, insbesondere solche mit einem Siedepunkt, bei normalem oder vermindertem Druck, von höchstens 60°C, wobei Methyl besonderen Vorzug hat. Bei Verwendung eines solchen Derivats der Aldehyd-Komponente (II) arbeitet man auch in an sich bekannter Weise analog der oben für freies Aldehyd geschilderten Methode. Wenn man dann die Komponente (II) als Salz einer Base einsetzt, ist es vorteilhaft, das Reaktionsgemisch auf neutrale Reaktion einzustellen, z.B. durch Vorlegen der anderen Komponente (des N-Aminopiperazins III) in Form eines Säureadditionssalzes, oder aber durch vorsichtige Zugabe einer Säure, wie einer Carbonsäure, z.B. Essigsäure. Wenn man die Aldehyd-Komponente (II) in Form eines Derivats mit funktionell abgewandelter Aldehydgruppe einsetzt, so kann man zweckmässig in einem grösseren Ueberschuss der Hydrazin-Komponente (III), welche dann zugleich als Lösungsmittel dient, arbeiten. Die Reaktionsbedingungen, vor allem Temperatur und Druck, werden dabei so eingestellt, dass die flüchtigen Reaktionsprodukte (z.B. das Amin bzw. Hydrazin der Formel $ZH_2$), die durch die Austauschreaktion aus dem Ausgangsstoff (II) freigesetzt werden, kontinuierlich aus dem Reaktionsgemisch durch Abdestillieren entfernt werden. Zweckmässig wird der Druck so vermindert, dass die Temperatur beim Abdestillieren nicht etwa 60°C, vorzugsweise nicht etwa 40°C übersteigt. Unter ähnlichen Bedingungen kann man aber auch den Austausch in einem inerten organischen Lösungsmittel, wie einem der oben erwähnten, z.B. Dimethylsulfoxid, durchführen.

Die als Ausgangsstoff verwendeten Verbindungen sind entweder bekannt oder durch gewöhnliche Standardverfahren der synthetischen organischen Chemie zugänglich. So können die N-substituierten N-Aminopiperazine der Formel III z.B. durch Nitrosieren (z.B. mittels in situ freigesetzter salpetriger Säure oder Stickstoffdioxid $N_2O_4$) des entsprechenden einfach substituierten Piperazins der Formel HW, worin W die obgenannte Bedeutung hat, und nachfolgende konventionelle Reduktion des gebildeten Nitrosamins, z.B. mittels eines komplexen Hydrids, wie insbesondere Lithiumaluminiumhydrid, oder durch katalytische Hydrierung erhalten werden.

Die monosubstituierten Piperazine der Formel HW, sofern sie nicht bereits bekannt sind, werden in an sich

bekannter allgemeiner Weise hergestellt, indem man Piperazin oder ein an einem seiner Stickstoffatome geschütztes Derivat davon mit einer äquimolaren Menge eines reaktiven funktionellen Derivats eines entsprechenden Benzylalkohols, z.B. einer Verbindung der oben definierten Formel V, worin Y und $R^1$ - $R^5$ die obgenannten Bedeutungen haben, umsetzt und die gegebenenfalls vorhandene N-Schutzgruppe abspaltet. Als N-Schutzgruppe können alle üblichen, insbesondere von der Peptid-Chemie bekannten Schutzgruppen, z.B. solvolytisch oder hydrogeno lytisch abspaltbare Schutzgruppen, wie tert-Butyloxycarbonyl (BOC) bzw. Benzyloxycarbonyl, verwendet werden, wobei ihre Abspaltung nach bekannten allgemeinen Verfahren erfolgt. Auch die Umsetzung mit dem Reagens V wird in einer allgemein bekannten Weise, z.B. wie unten bei Verfahrensvariante b) beschrieben, durchgeführt.

Die als Ausgangsstoff verwendeten 3-Formylrifamycin-Verbindungen sind bereits bekannt, vgl. z.B. das erwähnte U.S. Patent 3,342,810, oder nach bekannten, z.B. in dieser Patentschrift beschriebenen, allgemeinen Verfahren zugänglich. In dieser Weise wird z.B. vorteilhaft die 3-Formylgruppe in Verbindungen mit bereits teilsweise gesättigter Grundstruktur, d.h. in die entsprechenden Tetrahydro-und Hexahydro-rifamycin-Derivate eingeführt. Diese wiederum erhält man z.B. aus entsprechenden bekannten 3-Aminomethylrifamycinen durch Sättigung der Doppelbindungen, welche in an sich bekannter Weise, vornehmlich unter allgemein bekannten Reaktionsbedingungen der katalytischen Hydrierung bei Anwendung von konventionellen Hydrierungsmitteln erfolgt. Dabei arbeitet man mit Wasserstoffgas bei normalem oder erhöhtem Druck unter Bedingungen der heterogenen oder homogenen Katalyse. Als Katalysatore für die erstere sind fein verteilte Metalle, z.B. Raney-Metalle, wie Raney-Nickel, oder Edelmetalle, wie Palladium, Platin oder Rhodium, welche gegebenenfalls auf einem Träger, wie Calciumcarbonat oder Bariumsulphat, verteilt sind, besonders gut geeignet. Für die homogene Katalyse verwendet man insbesondere komplexe Rhodiumverbindungen z.B. Tris-(triphenylphosphin)-rhodium(I)chlorid. Die Bedingungen kann man so modifizieren, dass die weniger reaktive isolierte 28,29-Doppelbindung nicht mitreduziert wird, indem man z.B. die Hydrierung beim Verbrauch von 2 Aequivalenten Wasserstoff unterbricht und das resultierende 16,17,18,19-Tetrahydroderivat isoliert; vornehmlich kann man zu diesem Zweck einen milderen Katalysator, wie z.B. Palladium an einem Träger, z.B. Aktivkohle oder Calciumcarbonat, verwenden, wobei die Reaktion unter Normaldruck und Raumtemperatur spontan beim Verbrauch von 2 Aequivalenten zum Stillstand kommt. Bei Anwendung von stärkeren Katalysatoren z.B. Platin, insbesondere in der in situ aus Platinoxid reduzierter Form, kann man die Hydrierung zu Sättigung aller 3 Doppelbindungen fortsetzen, was unter üblichen Bedingungen zum spontanen Stillstand der Hydrierung und zur Bildung vom entsprechenden 16,17,18,19,28,29-Hexahydroderivat führt.

Ihrer Natur nach führt die Hydrierung zur Entstehung eines Asymmetrie-Zentrums am C(16) und somit Gemisch von Epimeren, die sich voneinander durch die sterische Anordnung des am C(16) situierten C(30)-Methyl unterscheiden. Infolge der äusserst schwierigen und verlustreichen Trennung der Epimere durch physikalische Methoden wird üblicherweise das erhaltene Epimerengemisch als einheitliches Verfahrensprodukt betrachtet und verwendet. - Da die teilweise gesättigten Ausgangsstoffe in irgend einer Stufe ihrer Herstellung ein analoges Hydrieren ihrer ungesättigten Grundverbindungen benötigen, welches ein Epimerengemisch liefert, sind alle in dieser Beschreibung erwähnten 16,17,18,19-Tetrahydro- und 16,17,18,19,28,29-Hexahydro-rifamycin-Verbindungen allgemein, wenn nicht ausdrücklich anders angegeben, sinngemäss als ein Gemisch beider C(16)-epimeren Formen zu betrachten. -Die Raumanordnung der Doppelbindungen in 16,17-, 18,19- und 28,29-Stellung entspricht derjenigen von ungesättigten Rifamycinverbindungen des natürlichen Ursprungs.

In der Verfahrensvariante b) verwendet man als Reagens zur Einführung des Benzylrestes eine Verbindung der Formel V, worin Y z.B. für den Rest einer Halogen-, wie Chlor-, Brom- oder Iod-, -wasserstoffsäure, einer sauerstoffhaltigen anorganischen Säure, wie Schwefelsäure, Phosphorsäure, Kieselsäure, oder einer Halogensulfonsäure, wie Fluorsulfonsäure, oder einer organischen Sulfonsäure, wie einer aliphatischen oder aromatischen Sulfonsäure, z.B. einer Niederalkansulfonsäure oder einer gegebenenfalls, z.B. durch Niederalkyl oder Nitro, substituierten Benzolsulfonsäure steht. Y bedeutet insbesondere Chlor oder Brom, ferner Methansulfonyloxy oder p-Toluolsulfonyloxy.

Die Umsetzung erfolgt vorzugsweise in Anwesenheit einer Base, insbesondere eines stark basischen, nicht nucleophilen, tertiären Amins, insbesondere eines entsprechenden sterisch gehinderten aliphatischen und/oder araliphatischen Amins, wie Tri-niederalkyl-amins, z.B. Ethyl-diisopropylamins. Dabei wird die Rifamycin-Verbindung und das Alkylierungsmittel in äquimolaren Mengen verwendet, wobei auch die Base vorzugsweise im äquimolaren Verhältnis zugesetzt wird. Die Umsetzung wird üblicherweise in einer organischen Lösungsmittel, wie insbesondere einem Ether (z.B. einem der oben erwähnten) oder einem chlorierten aliphatischen Kohlen wasserstoff (z.B. Chloroform oder Dichlormethan), oder in zweckmässigen Gemischen davon durchgeführt. Die Reaktikonstemperatur beträgt üblicherweise 0°-70°; es wird beim atmosphärischen Druck gearbeitet.

Die Ausgangsstoffe der Formel V sind im allgemeinen bekannt oder können in Analogie zu den bekannten hergestellt werden, z.B. durch Halogenmethylieren eines entsprechend substituierten Benzolderivats oder durch Einführung der obgenannten Gruppe Y durch Austausch gegen Hydroxyl in einem entsprechend substituierten Benzylalkohol. Die Ausgangsstoffe der Formel IV werden durch die Kondensation der entsprechenden 3-Formylrifamycine der Formel II mit N-Aminopiperazin gemäss der oben geschilderten Verfahrensvariante a) erhalten.

Die Isolierung des Reaktionsproduktes aus einem verfahrensgemäss erhältlichen Reaktionsgemisch erfolgt in an sich bekannter Weise, z.B. durch Verdünnen mit Wasser, und/oder gegebenenfalls durch Neutralisieren

mit einer wässrigen Säure, wie einer anorganischen oder organischen Säure, z.B. einer Mineralsäure oder vorteilhaft Zitronensäure, und Zugabe eines mit Wasser nicht mischbaren Lösungsmittels, wie z.B. eines chlorierten Kohlenwasserstoffs, z.B. Chloroform oder Methylenchlorid, wobei das Reaktionsprodukt in die organische Phase übergeht, aus welcher es in üblicher Weise, z.B. durch Trocknen, Eindampfen des Lösungsmittels und Kristallisation und/oder Chromatographie des Rückstandes oder andere übliche Reinigungsmethoden in reiner Form erhalten werden kann.

Wie bereits oben erwähnt wurde, können die erhaltenen erfindungsgemässen Verfahrensprodukte in andere erfindungsgemässe Verfahrensprodukte übergeführt werden.

Da die Verfahrensprodukte sowohl in der Hydrochinonform der Formel I [Rif SV] wie in der Chinonform der Formel I [Rif S] oder, in Form eines Gemisches der beiden Formen erhalten werden, so kann man sie nachträglich in an sich bekannter Weise ineinander bzw. ein Gemisch der beiden in eine der beiden einheitlichen Formen überführen. Dabei kann die Umwandlung eines verfahrensgemäss erhältlichen Chinons der Formel I [Rif S] in das entsprechende Hydrochinon der Formel I [Rif SV] bzw. eines verfahrensgemäss erhältlichen Hydrochinons der Formel I [Rif SV] in ein Chinon der Formel I [Rif S], oder die Vereinheitlichung eines Gemisches der beiden Verbindungstypen mittels Reduktion bzw. Oxidation nach oder vorteilhafterweise vor der Isolierung des gewünschten Produkts durchgeführt werden. Die Reduktion kann durch Behandeln mit einem zur Reduktion eines Chinons in das entsprechende Hydrochinon geeigneten Reduktionsmittel, wie einem Alkalimetall-, z.B. Natrium-, -dithionit oder -hydrosulfit; Zink und Essigsäure, oder vorzugsweise mit Ascorbinsäure, die Oxidation durch Behandeln mit einem für die Umwandlung eines Hydrochinons in das entsprechende Chinon geeigneten Oxidationsmittel, wie Luftsauerstoff, Wasserstoffperoxid, Alkalimetall-, z.B. Kalium-, -ferricyanid, einem Persulfat, z.B. Ammoniumpersulfat, ferner auch Mangandioxid, bewirkt werden, wobei die Oxidation vorzugsweise unter basischen Bedingungen durchgeführt wird. Die Chinone sind meist violettrot bis schwarz gefärbte Verbindungen, während die Hydrochinone üblicherweise hell, z.B. gelb bis rot, gefärbt und besser kristallisierbar sind.

Die Verbindungen der vorliegenden Erfindung können Salze, insbesondere Säureadditionssalze und in erster Linie pharmazeutisch verwendbare Säureadditionssalze mit anorganischen oder organischen Säuren bilden; solche sind u.a. Halogen-, z.B. Chlor- und Bromwasserstoffsäure, Schwefelsäure, Phosphorsäure, Salpetersäure oder Perchlorsäure, oder aliphatische, alicyclische, aromatische oder heterocyclische Carbon-oder Sulfonsäuren, wie Ameisen-, Essig-, Propion-, Bernstein-, Glykol-, Milch-, Aepfel-, Wein-, Zitronen-, Fumar-, Malein-, Hydroxymalein-, Oxal-, Brenztrauben-, Phenylessig-, Benzoe-, p-Aminobenzoe-, Anthranil-, p-Hydroxybenzoe-, Salicyl-, p-Aminosalicylsäure, Embonsäure, Methansulfon-, Ethansulfon-, Hydroxyethansulfon-, Ethylendisulfon-, Halogenbenzolsulfon-, Toluolsulfon-, Naphthalensulfonsäuren oder Sulfanilsäure, ferner Methionin, Tryptophan, Lysin oder Arginin, sowie Ascorbinsäure. Hydrochinonverbindungen vom Typ der Formel I [Rif SV] können auch Salze mit Basen, z.B. Alkalimetall-, wie Natrium-, -salze bilden.

Die gewünschtenfalls durchzuführende Salzbildung und Freisetzen der Grundformen der Verbindungen der Formel I aus ihren Salzen erfolgt auch in an sich allgemein bekannter, konventioneller Weise. - So werden Hydrochinone der Formel I [Rif SV] in entsprechende Salze mit Basen, vor allem Alkalimetallsalze, durch Behandeln mit einer entsprechenden Base, insbesondere einer alkalisch reagierenden Verbindung, wie Hydroxid, Carbonat oder Bicarbonat, übergeführt; die Salze können in freie Hydrochinon-Verbindungen durch Ansäuern, z.B. mit anorganischen Säuren, wie insbesondere Halogenwasserstoffsäuren, umgewandelt werden. - Basisch reagierende Endstoffe, z.B. Chinone der Formel I [Rif S] können in ihre Säureadditionssalze z.B. durch Behandeln mit einer zur Salzbildung geeigneter Säure, wie einer der oben genannten, umgewandelt werden; umgekehrt wird durch Behandeln mit basisch reagierenden Mitteln, wie mit anorganischen Hydroxiden, Carbonaten und Bicarbonaten, oder organischen Basen und Ionenaustauschern eine solche basische Grundform einer Verbindung der Formel I [Rif S] freigesetzt.

Verbindungen der vorliegenden Erfindung können auch innere Salze, z.B. durch übliches acid-basisches Titrieren zum Neutralpunkt bzw. zum isoelektkrischen Punkt, bilden.

Die Salze der neuen Verbindungen können auch zur Reinigung der erhaltenen Verbindungen dienen, indem man die freien Verbindungen in Salze überführt, diese abtrennt und aus ihnen wiederum die freien Verbindungen gewinnt. Infolge der engen Beziehung zwischen den Verbindungen in freier Form und in Form ihrer Salze sind im vorausgegangenen und nachfolgend unter den freien Verbindungen sinn-und zweckgemäss gegebenenfalls auch die entsprechenden Salze zu verstehen.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates, z.B. Salzes, verwendet oder unter den Reaktionsbedingungen bildet.

Bei den Verfahren der vorliegenden Erfindung werden vorzugsweise solche Ausgangsstoffe verwendet, welche zu den eingangs als besonders wertvoll geschilderten Verbindungen führen.

Im Hinblick auf die oben beschriebenen pharmakologischen Eigenschaften der neuen Verbindungen umfasst die vorliegende Erfindung auch die Verwendung der erfindungsgemässen Wirkstoffe allein, gegebenenfalls zusammen mit Hilfsstoffen, oder in Kombination mit anderen Wirkstoffen, insbesondere (zur Herstellung von) Antibiotika oder Chemotherapeutika, als Mittel zur Behandlung von Krankheiten, bei denen, wie oben beschrieben worden ist, die reverse Transkriptase von Bedeutung ist, und zwar sowohl prophylaktisch, wie auch kurativ. Bei der Verwendung als Heilmittel werden die erfindungsgemässen Wirkstoffe in prophylaktisch bzw. kurativ wirksamen Mengen, vorzugsweise in Form von pharmazeutischen

Zusammensetzungen zusammen mit konventionellen pharmazeutischen Trägermaterialien oder Hilfsstoffen verabreicht. Dabei werden z.B. an Warmblüter mit einem Körpergewicht von etwa 70 kg je nach Spezies, Körpergewicht, Alter und individuellem Zustand, sowie je nach Applikationsweise und insbesondere auch je nach der jeweiligen Empfindlichkeit des Krankheitserregers, tägliche Dosen von etwa 50 bis 1000 mg die in akuten Fällen noch mehrfach überschritten werden dürfen, verabreicht. Singgemäss umfasst die Erfindung auch die entsprechende Methode zur medizinischen Behandlung von Warmblütern, vor allem Menschen.

Die Erfindung betrifft im weiteren pharmazeutische Zusammensetzungen, welche die Verbindungen der vorliegenden Erfindung als Wirkstoffe enthalten, sowie Verfahren zur Herstellung dieser Zusammensetzungen. Ebenfalls kann auch die gewerbsmässige Herrichtung der Wirkstoffe eingeschlossen sein.

Bei den erfindungsgemässen pharmazeutischen Zusammensetzungen handelt es sich z.B. um solche zur enteralen, wie peroralen oder rektalen, sowie zur parenteralen Verabreichung an Warmblüter. Entsprechende Dosiseinheitsformen, insbesondere zur peroralen Verabreichung, z.B. Dragées, Tabletten oder Kapseln, enthalten vorzugsweise von etwa 50 bis 500 mg, insbesondere von etwa 100 bis 300 mg des Wirkstoffs zusammen mit pharmazeutisch verwendbaren Träger- oder Hilfsstoffen.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister (unter Verwendung z.B. von Mais-, Weizen-, Reis- oder Kartoffelstärke), Gelatine, Traganth, Methylcellulose und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol. Dragée-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyethylenglykole und/oder Titandioxid enthalten, oder Lacklösungen in geeigneten organischen Lösungsmitteln oder Lösungsmittelgemischen, oder, zur Herstellung Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Lactose, Bindemitteln, wie Stärken und/oder Gleitmitteln, wie Talk oder Magnesiumstearat, und gegebenenfalls von Stabilisatoren enthalten. In weichen Kapseln ist der Wikstoff vorzugsweise in geeigneten Flüssigkeiten, wie fetten Oelen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole. Ferner können auch Gelatine-Rektalkapseln verwendet werden, die eine Kombination des Wirkstoffes mit einer Grundmasse enthalten; als Grundmassenstoffe kommen z.B. flüssige Triglyceride, Polyethylenglykole oder Paraffinkohlenwasserstoffe in Frage.

Zur parenteralen Verabreichung eignen sich in erster Linie wässrige Lösungen einer in Wasser löslichen Form des Wirkstoffes, z.B. eines wasserlöslichen Salzes, oder wässrige Injektionssuspensionen, welche viskositätserhöhende Stoffe, z.B. Natriumcarboxymethylcellulose, Sorbit und/oder Dextran und gegebenenfalls Stabilisatore enthalten. Dabei kann der Wirkstoff, gegebenenfalls zusammen mit Hilfsstoffen, auch in Form eines Lyophilisats vorliegen und vor der parenteralen Verabreichung durch Zugabe von geeigneten Lösungsmitteln in Lösung gebracht werden.

Die pharmazeutischen Zusammensetzungen der vorliegenden Erfindung können in an sich bekannter Weise, z.B. mittels konventioneller Misch-, Granulier-, Dragier-, Lösungs- oder Lyophilisierungsverfahren hergestellt werden. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Die nachfolgenden Beispiele illustrieren die oben beschriebene Erfindung, schränken jedoch deren Umfang in keiner Weise ein. Temperaturen werden in Celsiusgraden angegeben.

Beispiel 1: 3-Formyl-16,17,18,19-tetrahydrorifamycin SV

Zu einer Lösung von 2,4 g Tetrahydrorifamycin S in 25 ml THF (Tetrahydrofuran) wird nacheinander 1,21 g Mangandioxid, 1,5 ml tert-Butylazomethin und 0,34 ml tert-Butylamin zugegeben. Die violette Lösung wird 18 Stunden bei 50°C gerührt, anschliessend über Kieselgur filtriert, auf 15 ml konzentriert und mit 2,3 g Ascorbinsäure und 10 ml 16 %-iger wässriger Schwefelsäure versetzt. Die Lösung wird 3 Stunden bei 45°C gerührt, auf +5°C gekühlt, mit Wasser versetzt und bei pH 3,5 dreimal mit Ethylacetat extrahiert. Die organischen Phasen werden mit Wasser gewaschen, getrocknet und eingeengt. Chromatographie des Rohprodukts an Silicagel und Kristallisation aus Aceton-Ether-Petrolether ergibt 3-Formyl-16,17,18,19-tetrahydrorifamycin SV (16-Epimerengemisch) vom Smp. 215-220°C. Massenspektrum: $m/z = 729$ ($M^+$),

entsprechend der Summenformel $C_{38}H_{51}NO_{13}$. 360-MHz-$^1$H-NMR ($CDCl_3$): 10,5 ppm (-CH=O) [olefinische Protonen nach höherem Feld verschoben]; $^{13}$C-NMR ($CDCl_3$): 191,94 (-CH=O), 42,18 (C-16), 35,74 (C-19), 30,62 (C-18), 20,38 (C-17) ppm.

Beispiel 2: 3-Formyl-16,17,18,19,28,29-hexahydrorifamycin SV

In Analogie zu Beispiel 1 wird aus 4,5 g 16,17,18,19,28,29-Hexahydrorifamycin S in 50 ml THF, 2,25 g Mangandioxid, 2,8 ml tert-Butylazomethin, 1,2 ml tert-Butylamin, 1,9 g Ascorbinsäure und 20 ml 16%-iger wässriger $H_2SO_4$ 3-Formyl-16,17,18,19,28,29-hexahydrorifamycin SV (16-Epimerengemisch), Smp. 148-151°C hergestellt. Massenspektrum: m/z = 731, entsprechend der Summenformel $C_{38}H_{53}NO_{13}$. 360-MHz-$^1$H-NMR ($CD_3OD$): 10,50 ppm (-CH=O) [olefinische Protonen verschwunden]; $^{13}$C-NMR ($CD_3OD$): 194,60 (-CH=O), 66,49 (C-29), 44,48 (C-16), 36,16 (C-19), 32,50 (C-18), 31,02 (C-28), 22,97 (C-17) ppm.

Beispiel 3: 3-[4-(2,4,6-Trimethylbenzyl)-piperazinyliminomethyl]-rifamycin SV

Zu einer Lösung von 5 g 3-Formylrifamycin SV in 100 ml THF werden 2 g 1-Amino-4-(2,4,6-trimethylbenzyl)-piperazin zugesetzt. Die tiefrote Reaktionslösung wird 10 Minuten bei Raumtemperatur gerührt und anschliessend im Vakuum konzentriert. Das Konzentrat wird mit Wasser versetzt und bei pH 3,5 mit Methylenchlorid extrahiert. Die organische Phase wird über $Na_2SO_4$ getrocknet und eingedampft. Der Rückstand wird aus Ether-Hexan kristallisiert, wobei rote Kristalle vom Smp. 161-168°C (Zersetzung) erhalten werden.

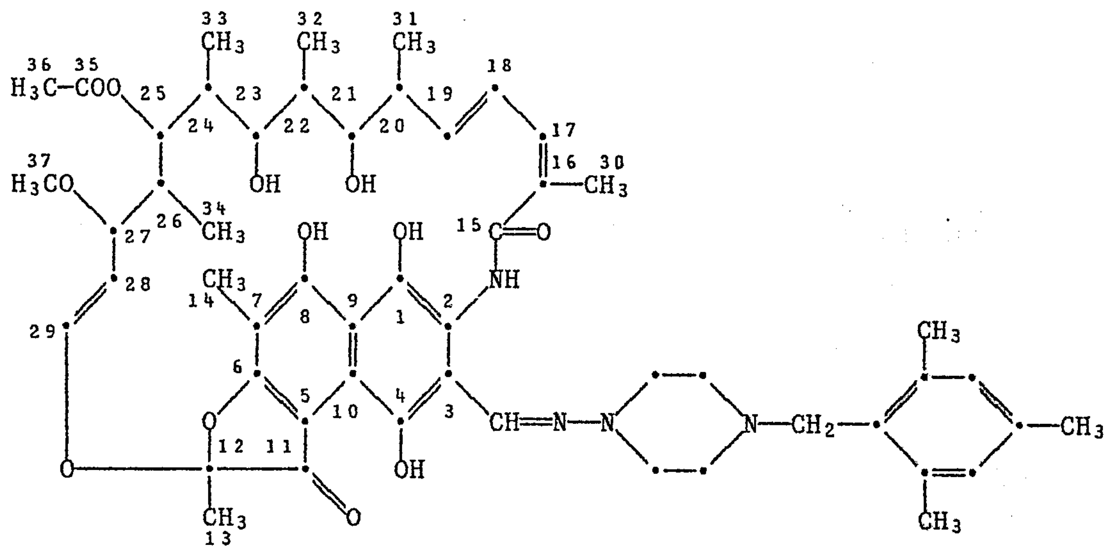

Massenspektrum: m/z = 939 (M-H)$^+$, entsprechend der Summenformel $C_{52}H_{68}N_4O_{12}$. $^{13}$C-NMR ($CDCl_3$): 134.70 (-C=N-N); 137.89 (2C), 136.52, 131.11, 128.95 (2C) (aromat. C); 56.95 (>N-COCHC$_2$); 51.31 (2C), 50.63 (2C) (piperazinyl-C) ppm.

A. Natriumsalz

Zur Herstellung des Natriumsalzes werden äquivalente Mengen 3-[4-(2,4,6-Trimethylbenzyl)-piperazinyliminomethyl]-rifamycin SV und Natriumhydrogencarbonat in einem Gemisch von Dioxan und Wasser gelöst und die Lösung lyophilisiert.

B. 3-[4-(2,4,6-Trimethylbenzyl)-piperazinyliminomethyl]-rifamycin S (Oxidation zum Chinon)

Eine Lösung von 2 g 3-[4-(2,4,6-Trimethylbenzyl)-piperazinyliminomethyl]-rifamycin SV in 50 ml Methylenchlorid wird mit 4 g pulverigem Mangandioxid bei Raumtemperatur intensiv gerührt, bis gemäss Dünnschichtchromatographie (DC) alles Ausgangsmaterial verschwunden ist. Die festen Anteile werden abfiltriert und das Filtrat zur Trockne eingedampft. Es resultieren 2 g der Titelverbindung der S-Reihe als amorpher blauschwarzer Feststoff ohne scharfen Schmelzpunkt.
Massenspektrum: m/z = 937 (M-H)$^+$ entsprechend der Summenformel $C_{52}H_{66}N_4O_{12}$.

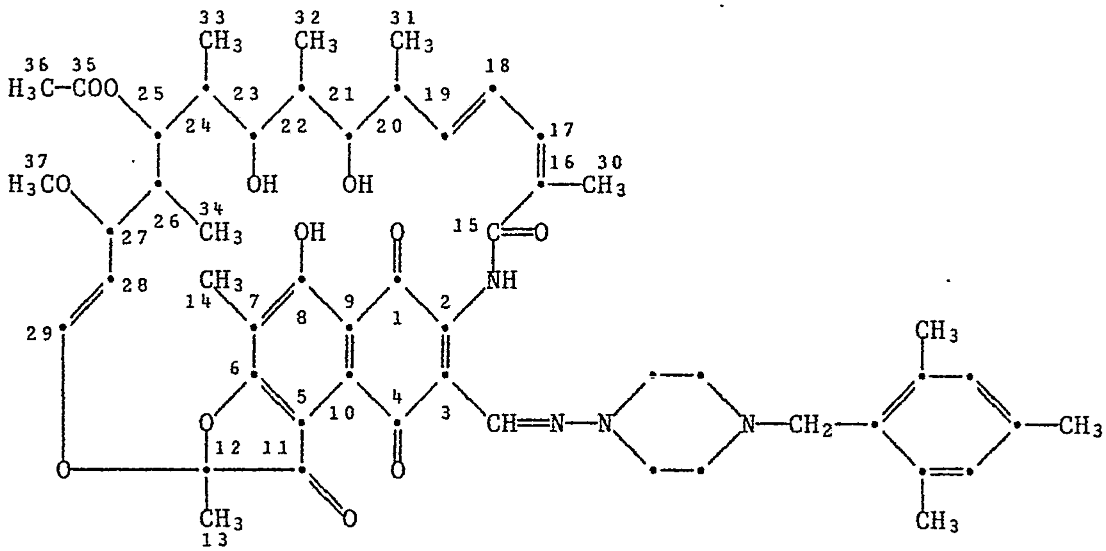

C. Herstellung des Ausgangsstoffes

Das als Ausgangsstoff verwendete 1-Amino-4-(2,4,6-trimethylbenzyl)-piperazin kann folgendermassen hergestellt werden:

a) Benzylierung von Piperazin

Zu einer Lösung von 25,3 g Piperazin-Hexahydrat in 100 ml Ethanol werden 20 g 2,4,6-Trimethylbenzylchlorid, gelöst in 50 ml Ethanol, zugegeben. Die Lösung wird über Nacht zur Rückflusstemperatur erhitzt. Nach Abkühlen wird der Alkohol im Vakuum entfernt. Der Rückstand wird in 100 ml 2N NaOH aufgeschlämmt und mit Methylenchlorid extrahiert Die vereinigten organischen Auszüge werden getrocknet und eingedampft. Der kristalline Rückstand (26 g) wird in Hochvakuum destilliert. Das ölige Destillat (12 g) erstarrt zu farblosen Kristallen vom N-(2,4,6-Trimethylbenzyl)-piperazin, Smp. 103-104°C.

Massenspektrum: m/z = 218 ($M^+$), entsprechend der Summenformel $C_{14}H_{22}N_2$.

b) Nitrosierung:

Eine wässrige Lösung von 5,86 g N-(2,4,6-Trimethylbenzyl)-piperazin wird bei 5° mit konzentrierter Salzsäure auf pH 1,1 gebracht und unter Kühlung auf 0-5° mit einer Lösung von 3,18 g Natriumnitrit in 5 ml Wasser tropfenweise behandelt, weitere 2 Stunden bei 5-10° gerührt und mit etwa 58 ml wässriger 2N-Lösung von Natronlauge auf pH 13 gestellt. Das Reaktionsgemisch wird mit 3 Portionen von jeweils 80 ml Ethylacetat extrahiert, und die vereinigten organischen Lösungen werden mit Natriumsulfat getrocknet und eingedampft. Der ölige Rückstand wird ohne Reinigung für die nächste Stufe verwendet.

c) Reduktion:

Zu einer gerührten Suspension von 1,71 g Lithiumaluminiumhydrid in 150 ml Tetrahydrofuran wird bei Rückflusstemperatur eine Lösung von 6,76 g rohem 1-Nitroso-4-(2,4,6-trimethylbenzyl)-piperazin (hergestellt gemäss Stufe b) in 30 ml Tetrahydrofuran zugetropft. Das Reaktionsgemisch wird weitere 3 Stunden bei Raumtemperatur gerührt, unter Eiskühlung mit 10 ml wässriger 2N-Natronlauge-Lösung und nachher mit 10 ml Wasser versetzt und weitere 2 Stunden bei Raumtemperatur gerührt. Der Niederschlag wird durch Abnutschen entfernt und auf der Nutsche mit heissem Isopropylalkohol nachgewaschen. Das Filtrat (samt den Isopropylalkohol-Anteilen), im Vakuum eingedampft, ergibt das gewünschte 1-Amino-4-(2,4,6-trimethylbenzyl)-piperazin als leicht gelb gefärbtes Oel in einer für die oben beschriebene Hydrazon-Bildung ausreichenden Reinheit.

In analoger Weise können durch die Verfahrensstufen a-c, ausgehend vom entsprechenden Halogenid (Chlorid oder Bromid), auch die folgenden 4-substituierten N-Amino-piperazine hergestellt und als Rohprodukt weiter eingesetzt werden.

1-Amino-4-(1-naphthylmethyl)-piperazin; 1-Amino-4-(2,6-dimethyl-4-tert-butylbenzyl)-piperazin; 1-Amino-4-(4-biphenylylmethyl)-piperazin; 1-Amino-4-(4-tert-butylbenzyl)-piperazin; 1-Amino-4-(9-anthrylmethyl)-piperazin; 1-Amino-4-(2,3-dimethylbenzyl)-piperazin; 1-Amino-4-(2,4-dimethylbenzyl)-piperazin; 1-Amino--4-(2,5-dimethylbenzyl)-piperazin; 1-Amino-4-(2,6-dimethylbenzyl)-piperazin; 1-Amino-4-(3,4-dimethylbenzyl)-piperazin sowie 1-Amino-4-(3,5-dimethylbenzyl)-piperazin.

Beispiel 4: 3-[4-(2,4,6-Trimethylbenzyl)-piperazinyliminomethyl]-16,17,18,19-tetrahydro-rifamycin SV

Zu einer Lösung von 0,3 g 3-Formyl-16,17,18,19-tetrahydro-rifamycin SV in 20 ml THF werden 0,12 g 1-Amino-4-(2,4,6-trimethylbenzyl)-piperazin zugegeben. Die tiefrote Reaktionslösung wird 10 Minuten bei Raumtemperatur gerührt und anschliessend konzentriert. Das Konzentrat wird mit Wasser versetzt und bei pH 3,5 mit Methylenchlorid extrahiert. Die organische Phase wird mit $Na_2SO_4$ getrocknet und eingedampft. Der Rückstand wird aus Ether-Petrolether kristallisiert. Die erhaltene Titelverbindung zersetzt sich bei Schmelzpunktbestimmung in breiten Temperaturgrenzen oberhalb 100°.

Massenspektrum: m/z = 943 $(M-H)^+$, entsprechend der Summenformel $C_{52}H_{72}N_4O_{12}$. 360-MHz-NMR ($CDCl_3$): 8,17 (CH=N); 3,53 ($N-CH_2$); olefinische H-17, 18, 19 abwesend, 6,85 (2 aromat. Protonen) ppm. $^{13}C$-NMR ($CDCl_3$): 134.12 (CH=N); 138.04 (2C), 136,70, 131.19, 129.11 (2C) (aromat. C); 57.07 ($N-CH_2$); 51.59 (2C); 51.26 (2C) (piperidyl-C); 43.85 (C-16); 36.53 (C-19); 31.40 (C-18); 21.45 (C-17) ppm.

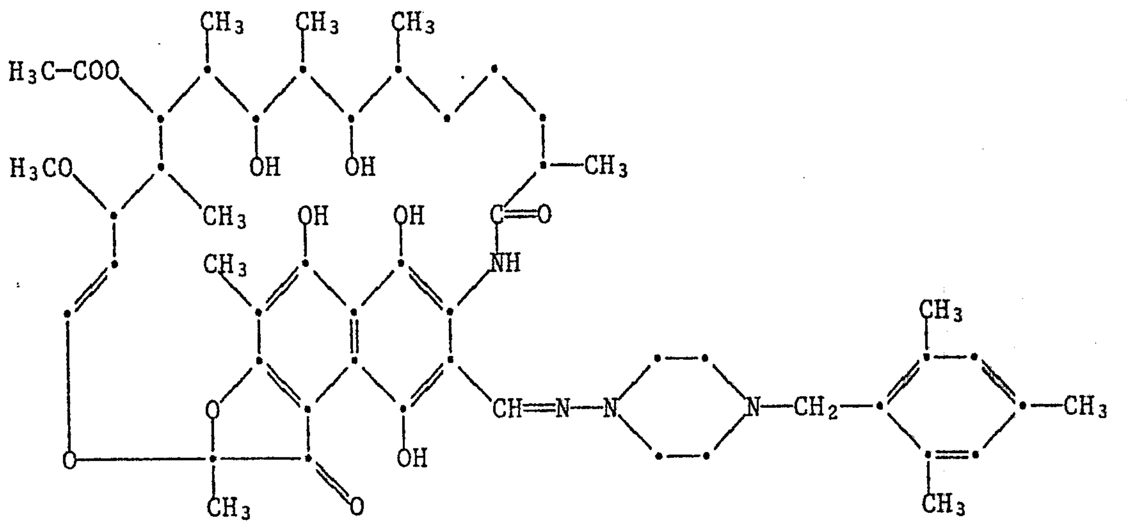

A. Natriumsalz

Zur Herstellung des Natriumsalzes werden äquivalente Mengen 3-[4-(2,4,6-Trimethylbenzyl)-piperazinyliminomethyl]-16,17,18,19-tetrahydrorifamycin SV und Natriumhydrogencarbonat in einem Gemisch von Dioxan und Wasser gelöst, und die Lösung lyophylisiert.

B. 3-[4-(2,4,6-Trimethylbenzyl)-piperazinyliminomethyl]-16,17,18,19-tetrahydro-rifamycin S (Oxidation zum Chinon)

Eine Lösung von 2 g der im Hauptbeispiel gewonnenen Hydrochinon-Verbindung in 50 ml Methanol wird mit 10 ml einer 10 %-Lösung von Ferricyanid in 10 %-Natriumbicarbonat-Lösung versetzt und 5 Minuten intensiv gerührt. Anschliessend wird vorsichtig mit 10 %-Zitronensäure-Lösung auf pH 3,5 angesäuert und die wässrige Phase dreimal mit Methylenchlorid extrahiert. Die organische Phase wird getrocknet und eingedampft. Es resultieren 2 g der Titelverbindung als blauschwarzer Feststoff ohne scharfen Schmelzpunkt. Massenspektrum: m/z = 941 $(M-H)^+$ entsprechend der Summenformel $C_{52}H_{70}N_4O_{12}$.

Beispiel 5: 3-[4-(2,4,6-Trimethylbenzyl)-piperazinyliminomethyl]-16,17,18,19,28,29-hexahydro-rifamycin SV

Zu einer Lösung von 0,3 g 3-Formyl-16,17,18,19,28,29-hexahydrorifamycin SV in 20 ml THF werden 0,12 g 1-Amino-4-(2,4,6-trimethylbenzyl)-piperazin zugesetzt. Die tiefrote Reaktionslösung wird 30 Minuten bei Raumtemperatur gerührt und im Vakuum konzentriert. Das Konzentrat wird mit Wasser versetzt und bei pH 3,5 mit Methylenchlorid extrahiert. Die organische Phase wird über $Na_2SO_4$ getrocknet und eingedampft. Der Rückstand wird aus Ether-Petrolether kristallisiert; die resultierende Titelverbindung schmilzt mit Zersetzung im breiten Temperaturbereich oberhalb 125°.

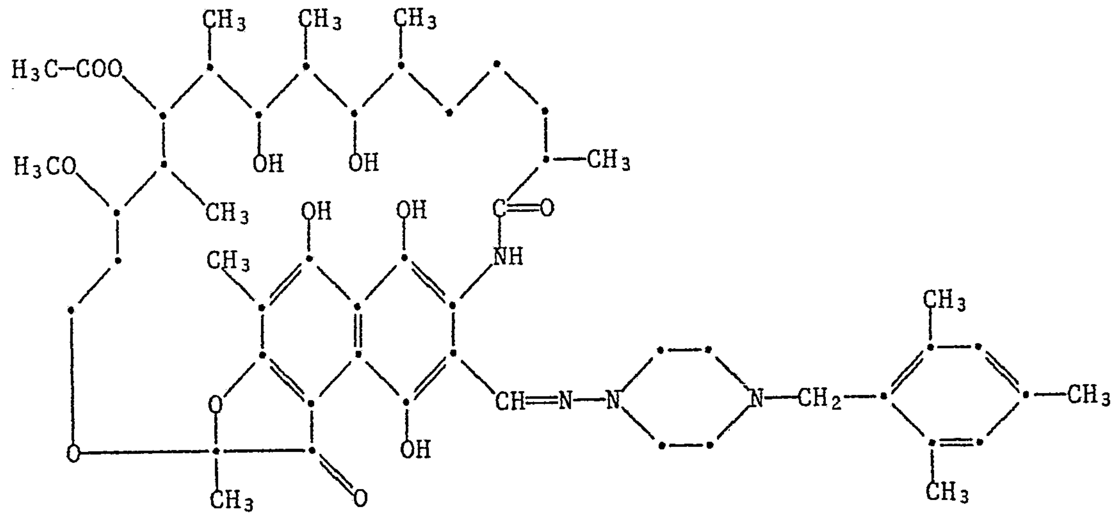

Massenspektrum: m/z = 945 $(M-H)^+$, entsprechend der Summenformel $C_{52}H_{74}N_4O_{12}$. 360-MHz-NMR ($CDCl_3$): 8,20 (CH=N-), 8,85 (2 aromat. Protonen), 3.55 (N-$CH_2$-), olefinische H-17,18,19,28,29 fehlen, ppm.

$^{13}$C-NMR ($CDCl_3$): 134.64 (-CH=N); 138.66 (2C), 137.31, 131.77, 129.62 (2C) (aromat. C); 56.04 (N-$CH_2$-); 52.19 (2C), 51.78 (2C) (piperidyl-C); 65.91 (C-29); 44.49 (C-16; 37.05 (C-19); 31.90 (C-18); 30.12 (C-28); 22.79 (C-17) ppm.

Natriumsalz

Zur Herstellung des Natriumsalzes werden äquivalente Mengen der Titelverbindung und Natriumhydrogencarbonat in einem Gemisch von Dioxan und Wasser gelöst, und die Lösung lyophilisiert.

Beispiel 6: 3-[4-(4-Biphenylylmethyl)-piperazinyliminomethyl]-rifamycin SV

Zu einer Lösung von 1 g 3-Formylrifamycin SV in 30 ml THF werden 0,5 g 1-Amino-4-(4-biphenylylmethyl)-piperazin zugefügt. Die tiefrote Reaktionslösung wird 10 Minuten bei Raumtemperatur gerührt und im Vakuum konzentriert. Das Konzentrat wird mit Wasser versetzt und bei pH 3,5 mit Methylenchlorid extrahiert. Die organische Phase wird mit $Na_2SO_4$ getrocknet und eingedampft. Der Rückstand wird aus Aceton-Ether-Hexan kristal lisiert, wobei rote Kristalle vom Smp. 164-167° (Zersetzung) erhalten werden. Massenspektrum: m/z = 974 ($M^+$), entsprechend der Summenformel $C_{55}H_{66}N_4O_{12}$. 360-MHz-$^1$H-NMR ($CDCl_3$): 8,21 (-CH=N) 7,3-7,7 (aromat. Proton);
3,62 ( >N-$CH_2$-) ppm. $^{13}$C-NMR ($CDCl_3$): 134.28 (CH=N-); 140,82, 140,31, 136.83, 129.41 (2C), 128,77 (2C), 127,29, 127,06 (4C) (aromat. C); 62.26 ($CH_2$-N<); 51.82 (2C), 50.45 (2C) (piperidyl-C) ppm.

Natriumsalz

Zur Herstellung der Natriumsalz werden äquivalente Mengen der Titelverbindung und Natriumhydrogencarbonat in einem Gemisch von Dioxan und $H_2O$ gelöst, und die Lösung lyophilisiert.

Beispiel 7: 3-[4-(4-Biphenylylmethyl)-piperazinyliminomethyl]-16,17,18,19-tetrahydro-rifamycin SV

Zu einer Lösung von 0,1 g 3-Formyl-16,17,18,19-tetrahydrorifamycin SV in 10 ml THF werden 54 mg 1-Amino-4-(4-biphenylylmethyl)-piperazin zugegeben. Die tiefrote Reaktionslösung wird 16 Minuten bei Raumtemperatur gerührt und im Vakuum konzentriert. Das Konzentrat wird mit Wasser versetzt und bei pH 3,5 mit Methylenchlorid extrahiert. Die organische Phase wird mit $Na_2SO_4$ getrocknet und eingedampft. Der Rückstand wird aus Ether-Hexan gefällt. Die erhaltene amorphe Titelverbindung hat folgende physikalische Daten:
Massenspektrum: m/z = 978 ($M^+$), entsprechend der Summenformel $C_{55}H_{70}N_4O_{12}$, 360-MHz-NMR ($CDCl_3$); 8,20 (-CH=N); 7,3-7,7 (aromat. Protonen); 3.65 (N-$CH_2$) ppm; olefinische 17,18,19-Protonen fehlen. $^{13}$C-NMR ($CDCl_3$): 134.20 (-CH=N-); 140.85, 140.36, 136.90, 129,40 (2C), 128.80 (2C), 127.30, 127.10 (4C) (aromat. C); 62.24 ( >N-$CH_2$); 52.10 (2C), 50.98 (2C) (piperazinyl-C); 43.93 (C-16); 36.59 (C-19); 31.48 (C-18); 21.47 (C-17) ppm.

Natriumsalz

Zur Herstellung des Natriumsalzes werden äquivalente Mengen der Titelverbindung und Natriumhydrogencarbonat in einem Gemisch von Dioxan und Wasser gelöst, und die Lösung lyophilisiert.

Beispiel 8: 3-[4-(4-Biphenylylmethyl)-piperazinyliminomethyl]-16,17,18,19,28,29-hexahydro-rifamycin SV

Zu einer Lösung von 0,1 g 3-Formyl-16,17,18,19,28,29-hexahydrorifamycin SV in 10 ml THF werden 54 mg 1-Amino-4-(4-biphenylylmethyl)-piperazin zugefügt. Die tiefrote Reaktionslösung wird 10 Minuten bei Raumtemperatur gerührt und im Vakuum konzentriert. Das Konzentrat wird mit Wasser versetzt und bei pH 3,5 mit Methylenchlorid extrahiert. Die organische Phase wird mit $Na_2SO_4$ getrocknet und eingedampft. Der Rückstand wird aus Ether-Hexan gefällt, womit die Titelverbindung in amorpher Form resultiert.
Massenspektrum: m/z = 980 ($M^+$), entsprechend der Summenformel $C_{55}H_{72}N_4O_{12}$, 360-MHz-NMR ($CDCl_3$) 8,21 (C<u>H</u>=N-); 7,3-7,7 (aromat. Protone); 3.65 ($>N-CH_2$) ppm; olefinische H-17,18,19,28,29 fehlen. $^{13}C$-NMR ($CDCl_3$): 140.85, 140.31 (2C), 136.91, 129.42 (2C), 128.80 (2C), 127.50, 127.10 (4C) (aromat. C); 65.26 (C-29); 62.26 ($>N-\underline{C}H_2$); 52.18 (2C), 50.92 (2C) (piperazinyl-C); 43.94 (C-10): 36.49 (C-19); 31.40 (C-18); 29.57 (C-28); 22.28 (C-17) ppm.

Natriumsalz

Zur Herstellung des Natriumsalzes werden äquivalente Mengen der Titelverbindung und Natriumhydrogencarbonat in einem Gemisch von Dioxan und Wasser gelöst, und die Lösung lyophilisiert.

Beispiel 9: 3-[4-(2,6-Dimethyl-4-tert-butyl-benzyl)-piperazinyliminomethyl]-rifamycin SV

Zu einer Lösung vom 1 g 3-Formylrifamycin SV in 20 ml THF werden 0,94 g 1-Amino-4-(2,6-dimethyl-4-tert-butyl-benzyl)-piperazin gegeben. Die tiefrote Reaktionslösung wird 60 Minuten bei Raumtemperatur gerührt und zur Trockne eingedampft. Das Rohprodukt wird mit Wasser versetzt und bei pH 3,5 mit Methylenchlorid extrahiert. Die organische Phase wird mit $Na_2SO_4$ getrocknet und eingedampft. Der Rückstand wird aus Ether-Petrolether kristallisiert, wobei rote Kristalle der Titelverbindung vom Smp. 171-174° (Zersetzung) erhalten werden.
Massenspektrum: m/z = 982 ($M^+$), entsprechend der Summenformel $C_{55}H_{74}N_4O_{12}$, 360-MHz-$^1H$-NMR ($CDCl_3$); 8,20 (-C<u>H</u>=N); 7,04 (2H, arom.); 2.40 + 2.24 (2 aromat. $CH_3$), 1.32 (3 $CH_3$ von tert.-Butyl) ppm. $^{13}C$-NMR ($CDCl_3$): 134.00 (-<u>C</u>H=N); 149.81 (aromat. C); 137.51 (2C); 131.20, 125.15 (2C); 55.5 ($>N-\underline{C}H_2$); 51.35 (2C); 50.67 (2C) (piperazinyl-C); 34.13 (tert.-Butyl); 31.27 (3C, tert.-Butyl) ppm.

Natriumsalz

Zur Herstellung des Natriumsalzes werden äquivalente Mengen der Titelverbindung und Natriumhydrogencarbonat in einem Gemisch von Dioxan und Wasser gelöst, und die Lösung lyophilisiert.

Beispiel 10: 3-[4-(1-Naphthylmethyl)-piperazinyliminomethyl]-rifamycin SV

Zu einer Lösung von 5 g 3-Formylrifamycin SV in 100 ml THF werden 1,98 g 1-Amino-4-(1-naphthylmethyl)-piperazin zugegeben. Die tiefrote Reaktionslösung wird 10 Minuten bei Raumtemperatur gerührt und im Vakuum konzentriert. Das Konzentrat wird mit Wasser versetzt und bei pH 3,5 mit Methylenchlorid extrahiert. Die organische Phase wird mit $Na_2SO_4$ getrocknet und eingedampft. Der Rückstand wird aus Ether kristallisiert, wobei rote Kristalle der Titelverbindung vom Smp. 165-169° (Zersetzung) erhalten werden.
Massenspektrum: m/z = 947 $(M-H)^-$, entsprechend der Summenformel $C_{53}H_{64}N_4O_{12}$. $^{13}C$-NMR ($CDCl_3$); 134,75 (CH=N-); 133,84, 133.33, 132.42, 129.34, 128.38, 127.41, 125.70, 125.02, 124.62, 123.14 (10 Naphthyl-C); 60.77 ($N-\underline{C}H_2-$); 51.88 (2C), 50.34 (2C) (piperazinyl-C) ppm.

Natriumsalz

Zur Herstellung des Natriumsalzes werden äquivalente Mengen der Titelverbindung und Natriumhydrogencarbonat in einem Gemisch von Dioxan und Wasser gelöst, und die Lösung lyophilisiert.

Beispiel 11: 3-[4-(9-Anthrylmethyl)-piperazinyliminomethyl]-rifamycin SV

Zu einer Lösung von 1 g 3-Formylrifamycin SV in 20 ml THF werden 0,85 g 1-Amino-4-(9-anthrylmethyl)-piperazin zugegeben. Die tiefrote Reaktionslösung wird 90 Minuten bei Raumtemperatur gerührt und im Vakuum konzentriert. Das Konzentrat wird mit Wasser versetzt und bei pH 3,5 mit Methylenchlorid extrahiert. Die organische Phase wird mit $Na_2SO_4$ getrocknet und eingedampft. Der Rückstand wird aus Aceton-Ether-Hexan kristallisiert, wobei rote Kristalle der Titelverbindung vom Smp. 168-173° (Zersetzung) erhalten werden.
Massenspektrum: m/z = 998 ($M^+$), entsprechend der Summenformel $C_{57}H_{66}N_4O_{12}$, 360-MHz-$^1H$-NMR ($CDCl_3$); 8.37 (-C<u>H</u>=N-); 7.5 + 8.0 (aromat. Protonen); 4,45 ($N-CH_2-$) ppm. $^{13}C$-NMR($CDCl_3$): 133.89 (-<u>C</u>H=N-); 131.13 (2C), 131.06 (2C), 129.06 (2C), 128.80 (2C), 127.63, 125,47 (2C), 124.63 (2C), 124.54 (2C) (aromat. C); 53.52 ($N-\underline{C}H_2-$); 51.51 (2C), 50.29 (2C) (piperazinyl-C) ppm.

Natriumsalz

Zur Herstellung des Natriumsalzes werden äquivalente Mengen der Titelverbindung und Natriumhydrogencarbonat in einem Gemisch von Dioxan und Wasser gelöst, und die Lösung lyophilisiert.

Beispiel 12: 3-[4-(9-Anthrylmethyl)-piperazinyliminomethyl]-16,17,18,19-tetrahydro-rifamycin SV

Zu einer Lösung von 0,2 g 3-Formyl-16,17,18,19-tetrahydrorifamycin SV in 10 ml THF werden 160 mg 1-Amino-4-(9-anthrylmethyl)-piperazin zugefügt. Die tiefrote Reaktionslösung wird 10 Minuten bei Raumtemperatur gerührt und im Vakuum konzentriert. Das Konzentrat wird mit Wasser versetzt und bei pH 3,5 mit

Methylenchlorid extrahiert. Die organische Phase wird mit $Na_2SO_4$ getrocknet und eingedampft. Der Rückstand wird aus Ether-Petrolether gefällt, womit die Titelverbindung in amorpher Form resultiert. Massenspektrum: m/z = 1002 ($M^+$), entsprechend der Summenformel $C_{57}H_{70}N_4O_{12}$, 360-MHz-$^1$H-NMR ($CDCl_3$); 8.48 (-CH=N-); 4.52 (-$CH_2$) ppm; olefinische H-17, H-18, H-19 fehlen. $^{13}$C-NMR($CDCl_3$): 134.38 (-CH=N-); 131.46 (2C), 131.40 (2C), 129.09 (2C), 128.86, 127.82, 125,78 (2C), 124.95 (2C), 124.83 (2C) (aromat. C); 53.84 (N-$CH_2$-); 52.05 (2C), 51.17 (2C) (piperazinyl-C); 45.90 (C-16); 31.40 (C-17); 21.44 (C-18); 36.54 (C-19) ppm.

Natriumsalz

Zur Herstellung des Natriumsalzes werden äquivalente Mengen der Titelverbindung und Natriumhydrogencarbonat in einem Gemisch von Dioxan und Wasser gelöst, und die Lösung lyophilisiert.

Beispiel 13:

In Analogie zu Beispiel 3 werden durch Umsetzen von 3-Formyl-rifamycin SV mit entsprechenden am Phenylring substituierten 1-Amino-4-benzylpiperazinen die folgenden Hydrazone erhalten:
3-[4-(4-Methylbenzyl)-piperazinyliminomethyl]-rifamycin SV;
3-[4-(2,3-Dimethylbenzyl)-piperazinyliminomethyl]-rifamycin SV;
3-[4-(2,4-Dimethylbenzyl)-piperazinyliminomethyl]-rifamycin SV;
3-[4-(2,6-Dimethylbenzyl)-piperazinyliminomethyl]-rifamycin SV und
3-[4-(4-tert-Butylbenzyl)-piperazinyliminomethyl]-rifamycin SV.

Zur Herstellung eines entsprechenden Natriumsalzes löst man äquimolare Mengen einer dieser Verbindungen und Natriumbicarbonat in einem Gemisch von Dioxan und Wasser, und lyophilisiert die Lösung.

Beispiel 14:

In Analogie zu Beispiel 14 werden durch Umsetzen von 3-Formyl-16,17,18,19-tetrahydro-rifamycin SV mit entsprechenden am Phenylring substituierten 1-Amino-4-benzylpiperazinen die folgenden Hydrazone erhalten:
3-[4-(4-Methylbenzyl)-piperazinyliminomethyl]-16,17,18,19-tetrahydro-rifamycin SV;
3-[4-(2,3-Dimethylbenzyl)-piperazinyliminomethyl]-16,17,18,19-tetrahydro-rifamycin SV;
3-[4-(2,4-Dimethylbenzyl)-piperazinyliminomethyl]-16,17,18,19-tetrahydro-rifamycin SV;
3-[4-(2,6-Dimethylbenzyl)-piperazinyliminomethyl]-16,17,18,19-tetrahydro-rifamycin SV sowie
3-[4-(4-tert-Butylbenzyl)-piperazinyliminomethyl]-16,17,18,19-tetrahydro-rifamycin SV;

Zur Herstellung eines entsprechenden Natriumsalzes löst man äquimolare Mengen einer dieser Verbindungen und Natriumbicarbonat in einem Gemisch von Dioxan und Wasser, und lyophilisiert die Lösung.

Beispiel 15:

In Analogie zu Beispiel 5 werden durch Umsetzen von 3-Formyl-16,17,18,19,28,29-hexahydro-rifamycin SV mit entsprechenden am Phenylring substituierten 1-Amino-4-benzylpiperazinen die folgenden Hydrazone erhalten:
3-[4-(4-Methylbenzyl)-piperazinyliminomethyl]-16,17,18,19,28,29-hexahydro-rifamycin SV;
3-[4-(2,3-Dimethylbenzyl)-piperazinyliminomethyl]-16,17,18,19,28,29-hexahydro-rifamycin SV;
3-[4-(2,4-Dimethylbenzyl)-piperazinyliminomethyl]-16,17,18,19,28,29-hexahydro-rifamycin SV;
3-[4-(2,6-Dimethylbenzyl)-piperazinyliminomethyl]-16,17,18,19,28,29-hexahydro-rifamycin SV sowie
3-[4-(4-tert-Butylbenzyl)-piperazinyliminomethyl]-16,17,18,19,28,29-hexahydro-rifamycin SV.

Zur Herstellung eines entsprechenden Natriumsalzes löst man äquimolare Mengen einer dieser Verbindungen und Natriumbicarbonat in einem Gemisch von Dioxan und Wasser, und lyophilisiert die Lösung.

Beispiel 16:

Kapseln, enthaltend 250 mg 3-[4-(2,4,6-Trimethyl-benzyl)-piperazinyliminomethyl]-rifamycin SV, können wie folgt hergestellt werden:

Zusammensetzung (für 1000 Kapseln):
3-[4-(2,4,6-Trimethyl-benzyl)-piperazinyliminomethyl]-rifamycin SV 250,0 g
Maisstärke 50,0 g
Polyvinylpyrrolidon 15,0 g
Magnesiumstearat 5,0 g
Ethanol q.s.

Der Wirkstoff und die Maisstärke werden vermischt und mit einer Lösung des Polyvinylpyrrolidons in 50 g Ethanol befeuchtet. Die feuchte Masse wird durch ein Sieb mit einer Maschenweite von 3 mm gepresst und bei 45° getrocknet. Das trockene Granulat wird durch ein Sieb mit einer Maschen weite von 1 mm gesiebt und mit 5 g Magnesiumstearat vermischt. Die Mischung wird in Portionen von 0,320 g in Steckkapseln der Grösse 0 abgefüllt.

Alternative kann man auch die übrigen, gemäss Beispielen 3-15 hergestellten Verbindungen als Wirkungskomponente verwenden.

**Patentansprüche**

1. Eine Verbindung der Formel
Rif-CH=N-W (I)
worin Rif einen Rest der Teilformel

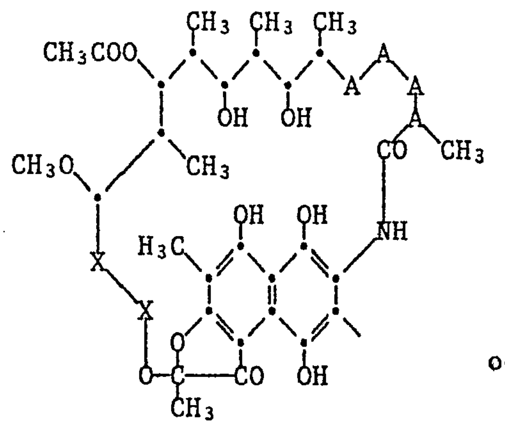

[Rif SV]

oder

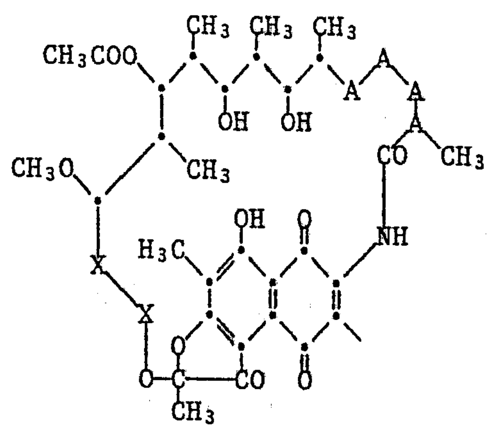

[Rif S]

darstellt, in welchem A-A-A-A Buta-1,3-dien-1,4-diyl und X-X Vinylen oder A-A-A-A Tetramethylen und X-X Ethylen oder Vinylen ist, und worin W einen Piperazinyl-Rest der Teilformel

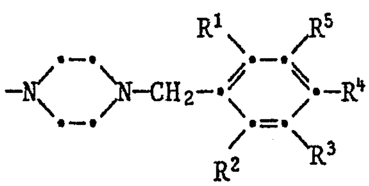

(W)

darstellt, worin jeder der Reste $R^1$, $R^2$, $R^3$, $R^4$ und $R^5$ zusammen mit seinem benachbarten Rest einen zweiwertigen aliphatischen Kohlenwasserstoffrest mit 3-10 C-Atomen bedeuten kann, welcher gemeinsam mit den entsprechenden 2 C-Atomen des zentralen Phenylrings einen anelierten 5-oder 6-gliedrigen carbocyclischen Ring bildet, wobei die übrigen Reste Wasserstoff oder $C_{1-4}$-Alkyl bedeuten, oder worin $R^4$ einen $C_{1-12}$-Alkyl, Phenyl oder Wasserstoff und $R^1$, $R^2$, $R^3$ und $R^5$ unabhängig je ein $C_{1-4}$-Alkyl oder Wasserstoff bedeuten, wobei mindestens einer aller Reste vom Wasserstoff verschieden sein muss, oder ein Salz davon.

2. Eine Verbindung gemäss Anspruch 1, worin in Formel I Rif die dort genannten Bedeutungen hat und in W jedes der Symbole $R^1$ und $R^2$ unabhängig je $C_{1-4}$-Alkyl, $R^3$ und $R^5$ unabhängig je Wasserstoff oder $C_{1-4}$-Alkyl und $R^4$ Phenyl oder $C_{1-12}$-Alkyl bedeuten, oder $R^2$ zusammen mit $R^3$ oder $R^3$ zusammen mit $R^4$ ein gegebenenfalls durch $C_{1-4}$-Alkyl substituiertes Buta-1,3-dien-1,4-diyl, Trimethylen oder Tetramethylen darstellen, $R^1$ und $R^5$ zusammen eine dieser Bedeutungen haben oder jedes einzeln Wasserstoff oder $C_{1-4}$-Alkyl bedeuten, wobei $R^4$ bzw. $R^2$ Wasserstoff oder $C_{1-4}$-Alkyl bedeutet, oder ein Salz davon.

3. Eine Verbindung gemäss Anspruch 1, worin in Formel I Rif die dort genannten Bedeutungen hat und in W jedes der Symbole $R^1$ und $R^2$ $C_{1-4}$-Alkyl, $R^4$ Wasserstoff, ein lineares $C_{6-12}$-Alkyl oder ein $C_{1-4}$-Alkyl, und $R^3$ und $R^5$ Wasserstoff bedeuten, oder ein Salz davon.

4. Eine Verbindung gemäss Anspruch 1, worin in Formel I Rif die dort genannten Bedeutungen hat und in W jedes der Symbole $R^1$, $R^2$, $R^3$ und $R^5$ Wasserstoff oder Methyl und $R^4$ Phenyl, Methyl oder tert-Butyl oder, falls mindestens einer der übrigen Reste Methyl ist, auch Wasserstoff bedeutet, oder ein Salz davon.

5. Eine Verbindung gemäss Anspruch 1, worin in Formel I Rif die dort angegebenen Bedeutungen hat und im W die Symbole $R^2$ und $R^3$ zusammen oder $R^3$ und $R^4$ zusammen Buta-1,3,-dien-1,4-diyl, $R^1$ und $R^5$ zusammen Buta-1,3-dien-1,4-diyl oder jedes einzeln Wasserstoff oder Methyl und $R^4$ bzw. $R^2$ Wasserstoff darstellen, oder ein Salz davon.

6. Eine Verbindung der Formel I gemäss einem der Ansprüche 1-5, worin Rif für einen der Reste [Rif SV] oder [Rif S] steht, in welchem A-A-A-A Buta-1,3-dien-1,4-diyl oder Tetramethylen und X-X Vinylen darstellt.

7. Eine Verbindung der Formel I gemäss einem der Ansprüche 1-6, worin Rif für [Rif SV] steht.

8. Eine Verbindung gemäss Anspruch 1, worin in Formel I Rif für [Rif SV] steht und W 4-(2,4,6-Trimethyl-benzyl)-piperazinyl bedeutet, oder ein Salz davon.

9. Eine Verbindung gemäss Anspruch 1, worin in Formel I Rif für [Rif SV] steht und W 4-(2,6-Dimethyl-4-tert-butyl-benzyl)-piperazinyl bedeutet, oder worin in Formel I Rif für [Rif SV] steht und W 4-(1-Naphthylmethyl)-piperazinyl bedeutet, oder worin in Formel I Rif für [Rif SV] steht und W 4-(9-Anthrylmethyl)-piperazinyl bedeutet, oder worin in Formel I Rif für [Rif SV] steht und W 4-(4-Biphenylylmethyl)-piperazinyl bedeutet, oder ein Salz davon.

10. Eine Verbindung gemäss einem der Ansprüche 1-9 in Form eines pharmazeutisch verwendbaren Salzes davon.

11. Eine Verbindung gemäss einem der Ansprüche 7-9 in Form eines pharmazeutisch verwendbaren Alkalimetallsalzes davon.

12. Eine Verbindung gemäss einem der Ansprüche 1-11 zur Verwendung als Hemmer der reversen Transkriptase.

13. Eine pharmazeutische Zusammensetzung enthaltend als Wirkstoff mindestens eine in einem der Ansprüche 1-12 definierten Verbindungen.

14. Verfahren zur Herstellung einer Verbindung der Formel I oder eines Salzes gemäss einem der Ansprüche 1-12 davon, dadurch gekennzeichnet, dass man

a) ein 3-Formylrifamycin der Formel

Rif-CH=Z (II),

worin Rif die im Anspruch 1 angegebene Bedeutung hat und Z eine freie oder funktionell abgewandelte Oxogruppe ist, mit einem N-Aminopiperazin der Formel

$W\text{-}NH_2$ (III),

worin W die im Anspruch 1 angegebenen Bedeutungen hat, umsetzt oder

b) ein vom N'-unsubstituierten Aminopiperazin abgeleitetes Hydrazon der Formel

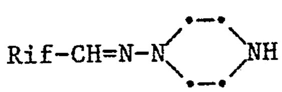

(IV),

worin Rif die im Anspruch 1 angegebenen Bedeutungen hat, mit einer Verbindung der Formel

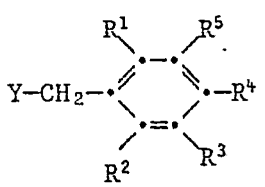

(V),

worin $R^1$, $R^2$, $R^3$, $R^4$ und $R^5$ die im Anspruch 1 angegebenen Bedeutungen haben und Y für den Rest einer starken anorganischen oder organischen Säure steht, umsetzt, und gewünschtenfalls, wenn eine Verbindung der Formel I in der Chinon-Form erwünscht ist, eine in der Hydrochinon-Form vorliegende Verbindung der Formel I mit einem Oxidationsmittel behandelt, und/oder, wenn eine Verbindung der Formel I in der Hydrochinon-Form erwünscht ist, eine in der Chinon-Form vorliegende Verbindung der Formel I mit einem Reduktionsmittel behandelt und/oder eine in freier Form vorliegende Verbindung der Formel I in ein Salz davon überführt oder eine freie Verbindung der Formel I aus einem Salz davon freisetzt.

15. Verfahren zur Herstellung einer pharmazeutischen Zusammensetzung enthaltend als Wirkstoff mindestens eine in einem der Ansprüche 1-15 definierte Verbindung, dadurch gekennzeichnet, dass man diesen Wirkstoff mit mindestens einem pharmazeutischen Trägermaterial verarbeitet.

16. Verwendung einer im Anspruch 1 definierten Verbindung zur Herstellung von pharmazeutischen Zusammensetzungen zur präventiven oder kurativen Behandlung von Krankheiten, bei welchen die reverse Transkriptase von Bedeutung ist.

Patentansprüche für die folgenden Vertragsstaaten: ES, GR

1. Verfahren zur Herstellung von Verbindungen der Formel

Rif-CH=N-W (I)

worin Rif einen Rest der Teilformel

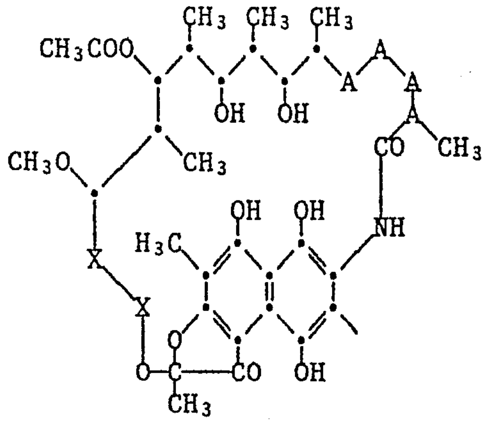

[Rif SV]

oder

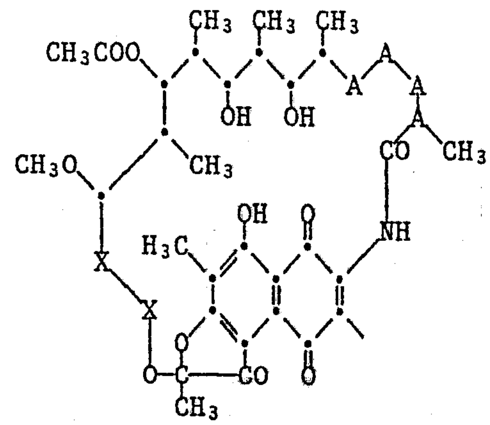

[Rif S]

darstellt, in welchem A-A-A-A Buta-1,3-dien-1,4-diyl und X-X Vinylen oder A-A-A-A Tetramethylen und X-X Ethylen oder Vinylen ist, und worin W einen Piperazinyl-Rest der Teilformel

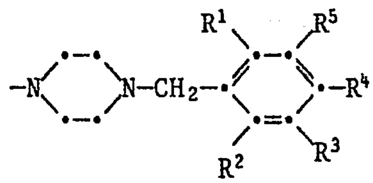

(W)

darstellt, worin jeder der Reste $R^1$, $R^2$, $R^3$, $R^4$ und $R^5$ zusammen mit seinem benachbarten Rest einen zweiwertigen aliphatischen Kohlenwasserstoffrest mit 3-10 C-Atomen bedeuten kann, welcher gemeinsam mit den entsprechenden 2 C-Atomen des zentralen Phenylrings einen anelierten 5-oder 6-gliedrigen carbocyclischen Ring bildet, wobei die übrigen Reste Wasserstoff oder $C_{1-4}$-Alkyl bedeuten, oder worin $R^4$ einen $C_{1-12}$-Alkyl, Phenyl oder Wasserstoff und $R^1$, $R^2$, $R^3$ und $R^5$ unabhängig je ein $C_{1-4}$-Alkyl oder Wasserstoff bedeuten, wobei mindestens einer aller Reste vom Wasserstoff verschieden sein muss, oder einem Salz davon, dadurch gekennzeichnet, dass man

a) ein 3-Formylrifamycin der Formel

Rif-CH=Z (II),

worin Rif die im Anspruch 1 angegebene Bedeutung hat und Z eine freie oder funktionell abgewandelte Oxogruppe ist, mit einem N-Aminopiperazin der Formel

$W-NH_2$ (III),

worin W die im Anspruch 1 angegebenen Bedeutungen hat, umsetzt oder

b) ein vom N'-unsubstituierten Aminopiperazin abgeleitetes Hydrazon der Formel

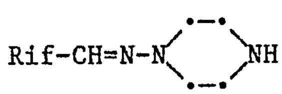

(IV),

worin Rif die im Anspruch 1 angegebenen Bedeutungen hat, mit einer Verbindung der Formel

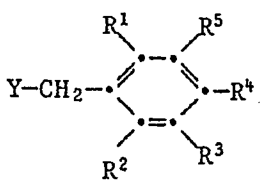

(V),

worin $R^1$, $R^2$, $R^3$, $R^4$ und $R^5$ die im Anspruch 1 angegebenen Bedeutungen haben und Y für den Rest einer starken anorganischen oder organischen Säure steht, umsetzt, und gewünschtenfalls, wenn eine Verbindung der Formel I in der Chinon-Form erwünscht ist, eine in der Hydrochinon-Form vorliegende Verbindung der Formel I mit einem Oxidationsmittel behandelt, und/oder, wenn eine Verbindung der Formel I in der Hydrochinon-Form erwünscht ist, eine in der Chinon-Form

vorliegende Verbindung der Formel I mit einem Reduktionsmittel behandelt und/oder eine in freier Form vorliegende Verbindung der Formel I in ein Salz davon überführt oder eine freie Verbindung der Formel I aus einem Salz davon freisetzt.

2. Verfahren gemäss Anspruch 1 zur Herstellung von Verbindungen der Formel I, worin Rif die in Anspruch 1 genannten Bedeutungen hat und in W jedes der Symbole $R^1$ und $R^2$ unabhängig je $C_{1-4}$-Alkyl, $R^3$ und $R^5$ unabhängig je Wasserstoff oder $C_{1-4}$-Alkyl und $R^4$ Phenyl oder $C_{1-12}$-Alkyl bedeuten, oder $R^2$ zusammen mit $R^3$ oder $R^3$ zusammen mit $R^4$ ein gegebenenfalls durch $C_{1-4}$-Alkyl substituiertes Buta-1,3-dien-1,4-diyl, Trimethylen oder Tetramethylen darstellen, $R^1$ und $R^5$ zusammen eine dieser Bedeutungen haben oder jedes einzeln Wasserstoff oder $C_{1-4}$-Alkyl bedeuten, wobei $R^4$ bzw. $R^2$ Wasserstoff oder $C_{1-4}$-Alkyl bedeutet, oder eines Salzes davon.

3. Verfahren gemäss Anspruch 1 zur Herstellung von Verbindungen der Formel I, worin Rif die in Anspruch 1 genannten Bedeutungen hat und in W jedes der Symbole $R^1$ und $R^2$ $C_{1-4}$-Alkyl, $R^4$ Wasserstoff, ein lineares $C_{5-12}$-Alkyl oder ein $C_{1-4}$-Alkyl, und $R^3$ und $R^5$ Wasserstoff bedeuten, oder eines Salzes davon.

4. Verfahren gemäss Anspruch 1 zur Herstellung von Verbindungen der Formel I, worin Rif die in Anspruch 1 genannten Bedeutungen hat und in W jedes der Symbole $R^1$, $R^2$, $R^3$ und $R^5$ Wasserstoff oder Methyl und $R^4$ Phenyl, Methyl oder tert-Butyl oder, falls mindestens einer der übrigen Reste Methyl ist, auch Wasserstoff bedeutet, oder eines Salzes davon.

5. Verfahren gemäss Anspruch 1 zur Herstellung von Verbindungen der Formel I, worin Rif die in Anspruch 1 angegebenen Bedeutungen hat und im W die Symbole $R^2$ und $R^3$ zusammen oder $R^3$ und $R^4$ zusammen Buta-1,3,-dien-1,4-diyl, $R^1$ und $R^5$ zusammen Buta-1,3-dien-1,4-diyl oder jedes einzeln Wasserstoff oder Methyl und $R^4$ bzw. $R^2$ Wasserstoff darstellen, oder eines Salzes davon.

6. Verfahren gemäss einem der Ansprüche 1-5 zur Herstellung von Verbindungen der Formel I, worin Rif für einen der Reste [Rif SV] oder [Rif S] steht, in welchem A-A-A-A Buta-1,3-dien-1,4-diyl oder Tetramethylen und X-X Vinylen darstellt.

7. Verfahren gemäss einem der Ansprüche 1-6 zur Herstellung von Verbindungen der Formel I, worin Rif für [Rif SV] steht.

8. Verfahren gemäss Anspruch 1 zur Herstellung der Verbindung der Formel I, worin Rif für [Rif SV] steht und W 4-(2,4,6-Trimethyl-benzyl)-piperazinyl bedeutet, oder eines Salzes davon.

9. Verfahren gemäss Anspruch 1 zur Herstellung der Verbindung der Formel I, worin Rif für [Rif SV] steht und W 4-(2,6-Dimethyl-4-tert-butyl-benzyl)-piperazinyl bedeutet, oder worin Rif für [Rif SV] steht und W 4-(1-Naphthylmethyl)-piperazinyl bedeutet, oder worin Rif für [Rif SV] steht und W 4-(9-Anthrylmethyl)-piperazinyl bedeutet, oder worin Rif für [Rif SV] steht und W 4-(4-Biphenylylmethyl)-piperazinyl bedeutet, oder eines Salzes davon.

10. Verfahren zur Herstellung einer pharmazeutischen Zusammensetzung enthaltend als Wirkstoff mindestens eine in einem der Ansprüche 1-9 definierte Verbindung, dadurch gekennzeichnet, dass man diesen Wirkstoff mit mindestens einem pharmazeutischen Trägermaterial verarbeitet.

Europäisches Patentamt

# EUROPÄISCHER RECHERCHENBERICHT

Nummer der Anmeldung

EP 88 81 0120

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DE-A-2 816 274 (DSO, PHARMACHIM) * Beispiel; Tabellen 1,2 * --- | 1-9,13, 14 | C 07 D 498/08<br>A 61 K 31/395//<br>(C 07 D 498/08<br>C 07 D 307:00<br>C 07 D 267:00 ) |
| Y | DD-A- 239 795 (CIBA-GEIGY AG) * Seiten 2,3, Abschnitt "Anwendungsgebiet der Erfindung"; Seite 5, Tabelle I * --- | 1-8,13 | |
| Y | DE-A-2 127 172 (GRUPPO LEPETIT S.P.A.) * Beispiel 3; Tabelle I, Seite 8 * --- | 1-8,13 | |
| A | THE JOURNAL OF ANTIBIOTICS, Band 24, Nr. 1, January 1971, Seiten 64-66; G. LANCINI et al.: "Antiviral activity of rifamycins and N-aminopiperazines" ----- | 1-7,13 | |

RECHERCHIERTE SACHGEBIETE (Int. Cl.4)

C 07 D 498/00

Der vorliegende Recherchenbericht wurde für alle Patentansprüche erstellt

| Recherchenort | Abschlußdatum der Recherche | Prüfer |
|---|---|---|
| BERLIN | 09-06-1988 | HASS C V F |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument